(19) Europäisches Patentamt
European Patent Office
Office européen des brevets

(11) **EP 4 745 233 A2**

(12) **EUROPEAN PATENT APPLICATION**

(43) Date of publication:
**20.05.2026 Bulletin 2026/21**

(21) Application number: **26168875.8**

(22) Date of filing: **12.02.2021**

(51) International Patent Classification (IPC):
***C12N 9/90*** (2006.01)

(52) Cooperative Patent Classification (CPC):
**C12P 21/00; C07K 14/245; C12N 9/1085;
C12N 9/90; C12N 15/63; C12N 15/70; C12P 7/42;
C12Y 205/01015; C12Y 504/0201**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.02.2020 EP 20157159**

(62) Document number(s) of the earlier application(s) in
accordance with Art. 76 EPC:
**21704286.0 / 4 103 719**

(71) Applicants:
• **Danmarks Tekniske Universitet
2800 Kongens Lyngby (DK)**
• **Enduro Genetics ApS
2200 Copenhagen N (DK)**

(72) Inventors:
• **Sommer, Morten Otto Alexander
2800 Kongens Lyngby (DK)**

• **Rugbjerg, Peter
2200 København N (DK)**
• **Pallisgaard Olsen, William Kristian
2800 Kongens Lyngby (DK)**
• **Tidemand Johansen, Anne Line
2200 København N (DK)**

(74) Representative: **Guardian
IP Consulting I/S
Diplomvej, Building 381
2800 Kgs. Lyngby (DK)**

Remarks:
•The complete document including Reference
Table(s) and the Sequence Listing(s) can be
downloaded from the EPO website
•This application was filed on 30-03-2026 as a
divisional application to the application mentioned
under INID code 62.

(54) **BURDEN-ADDICTED PRODUCTION STRAINS**

(57) The invention provides a microbial production cell for synthesis of a product, further comprising a burden-addiction genetic circuit whose expression confers a selective growth and/or survival advantage on those cells that synthesize the product; while limiting proliferation of low- or non-productive escaper cells.

**Figure 6**

**Description**

**FIELD OF THE INVENTION**

Burden-addicted production strains. The invention provides a microbial production cell for synthesis of a product, further comprising a burden-addiction genetic circuit whose expression confers a selective growth and/or survival advantage on those cells that synthesize the product; while limiting proliferation of non/low-productive escaper cells.

**BACKGROUND OF THE INVENTION**

An increasing share of the world's chemical production relies on microorganisms that are genetically engineered to function as cell factories, and tailor-made for the biosynthesis of a given molecule. Production processes, employing these cell-factories, are typically initiated from a starter culture of a small number of cells of a production organism, which go through a phase of growth and expansion of cell numbers in large fermentation tanks (up to 1,000,000 L volume). In some setups, production of a given molecule proceeds both during the growth phase and during a subsequent period (batch and fed-batch cultures); and alternatively, production may be continuous. A chemostat fermenter allows a production organism to be grown in a fermentation broth that is constantly diluted, thus tapping product and cells from the culture, while replenishing with fresh nutrient medium. On an industrial scale, such production processes may continue operation for 1-2 months before starting a new culture in a clean tank. The fermentation processes and equipment used in this industry are very similar, both for the production of a wide range of commodity small molecules and for therapeutic proteins, and consequently these processes are subject to similar problems. In other applications, microbial cells may be engineered to produce a non-native molecule that confers a commercial advantage in the environment in which the cell is cultured.

Cell factories engineered to allocate finite metabolic resources to the biosynthesis of a given molecule are subject of an unnatural load; commonly reflected in slower growth. This load places a selective pressure for cells unable to synthesize the product molecule (non-producing or lower-producing), this problem particularly arising when the production run is for extended periods of time (e.g. in chemostats). Such non-producing cells within an industrial fermentation are highly undesirable, since they consume nutrients, oxygen and space. Since lower- and non-producing cells grow faster, they have a strong selective advantage over producing cells. In a growing cell culture, such improvements in fitness can lead to significant out-competition of the producing cells over time. This drift from the optimal production state is an eventual reason for discarding the fermentation broth and spending resources on cleaning, sterilization, not to mention nutrients, to replenish the fermentation tank with new, producing organisms. Such non-producing cells originate from genetic mutations that arise in the cells of an original producing organism undergoing many growth divisions.

Since the occurrence of genetic mutations and non-genetic adaptation in cells of a production organism that lead to a decline in product formation by the cells during a production run, cannot be avoided, there is a need for methods for eliminating or slowing the growth of non-producing cells in the production. Preferably, such methods of elimination are sufficiently effective that they prevent the observed drift from the production state, and thereby prolong the life-time of an industrial fermentation.

The present invention addresses the problem of how to deprive non-producing members of a cell factory with a fitness advantage; such as to delay their proliferation amongst members of a productive cell factory over time, and thereby improve the productivity of the cell factory.

**SUMMARY OF THE INVENTION**

A first aspect of the invention provides a microbial production cell genetically engineered to synthesize a product, wherein said cell further comprises:

- an essential gene operably linked to a burden-sensing promoter,

wherein said promoter is heterologous with respect to said essential gene; wherein synthesis of the product confers a burden and/or fitness cost on said cell, and wherein expression of said essential gene is up-regulated when said burden-sensing promoter is induced by said burden and/or fitness cost relative to a basal level expression of said essential gene when said burden-sensing promoter is not induced.

A second aspect of the invention provides a method of product biosynthesis comprising the steps of:

- providing a microbial production cell of the invention,

- introducing the cell into a cultivation medium comprising substrate for production of said product,

- recovering said product.

A third aspect of the invention provides for a use of an essential gene operably linked to a burden-sensing promoter to enhance product yield of a cultured population of microbial production cells arising from a single cell, wherein said promoter is heterologous with respect to said essential gene; wherein production of the product confers a burden on said cell, and wherein expression of said essential gene is up-regulated when said burden-sensing promoter is induced by said burden relative to a basal level expression of said essential gene when said burden-sensing promoter is not induced.

A fourth aspect of the invention provides for a use of a microbial production cell of the invention for producing a biosynthetic product.

## DESCRIPTION OF THE INVENTION

### Brief description of the figures:

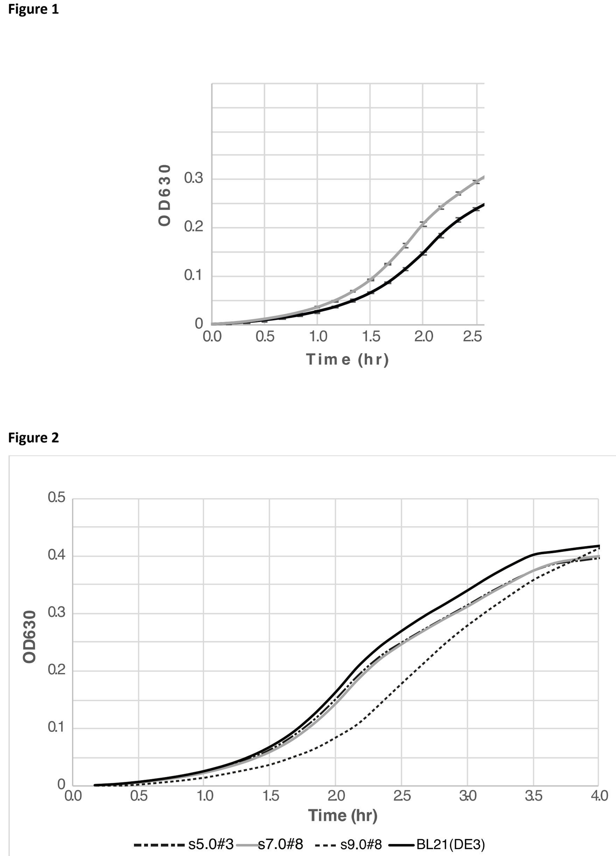

**Figure 1:** Graph showing growth (measured as optical density: OD $630_{nm}$) over time of cells of the parent strain *E. coli* BL21(DE3) (grey line) and cells of the parent strain transformed with a hGH-GFP production plasmid pEG34 (black line; pEG34). Growth of four replicate cultures of each strain was measured under hGH-GFP expression inducing conditions; error bars depict standard error of the mean (n = 4).

**Figure 2:** Graph showing growth (measured as optical density: OD $630_{nm}$) over time of cells of the parent strain *E. coli* BL21(DE3) (black line) and cells of the non-producing burden-addicted *E. coli* strains s5.0#3, s7.0#8 and s9.0#8 derived from the parent strain (as indicated; wherein essential genes *folP-glmM* are controlled by $p_{yccV}$, $p_{ycjX}$ and $p_{mutM}$ respectively). The measured growth rate is based on an average of four replicate cultures (n = 4).

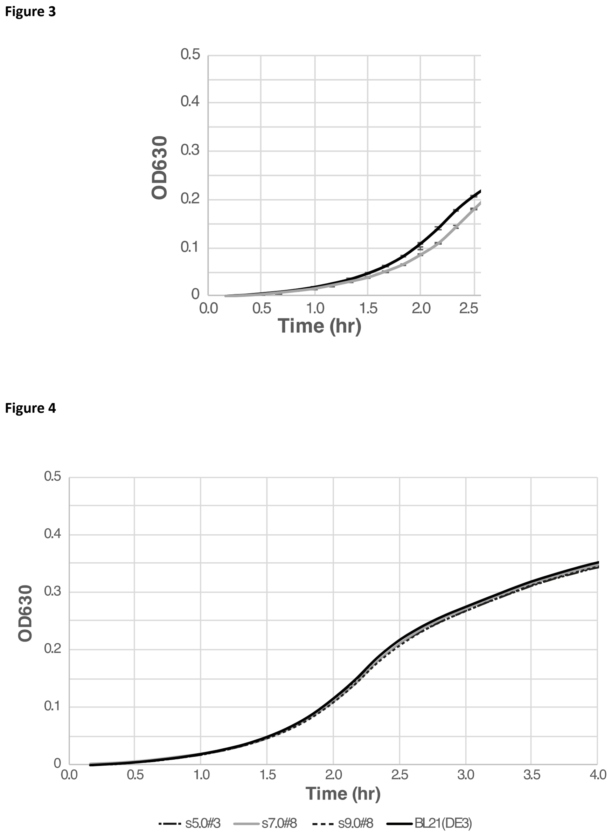

**Figure 3:** Graph showing growth (measured as optical density: OD $630_{nm}$) over time of cells of the burden-addicted strain s9.0#8 harboring either the hGH-GFP production plasmid pEG34 (black line) or the control plasmid pEG0 (grey line). Growth of four replicate cultures of each strain was measured under hGH-GFP expression inducing conditions; error bars depict standard error of the mean (n = 4).

**Figure 4:** Graph showing growth (measured as optical density: OD $630_{nm}$) over time of cells of the parent strain *E. coli* BL21(DE3) (black line), and cells of three burden-addicted strains: s5.0#3, s7.0#8 and s9.0#8 harboring and expressing the hGH-GFP production plasmid pEG34 (as indicated). The measured growth rate, measured under hGH-GFP expression inducing conditions, is based on an average of four replicate cultures (n = 4).

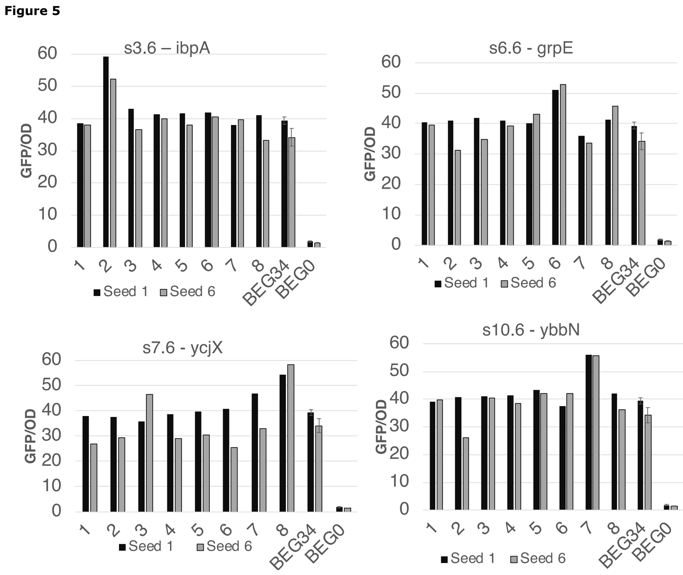

**Figure 5:** Histogram of GFP/OD values (Y-axis) measured during the first and sixth culture transfer (seed 1 and 6) of cultures of the hGH-GFP producing strains: s3.6 ($p_{ibpA}$), s6.6 ($p_{grpE}$), s7.6 ($p_{ycjX}$), and s10.6 ($p_{ybbN}$) comprising the respective burden-sensing promoters and one of four variant RBS coding sequences, driving expression of the essential gene, *folP-glmM*. Cultures of non-burden addicted *E. coli* strain BEG34 and the parent strain *E. coli* BL21(DE3), BEG0, are included as controls. Measurements are performed on cultures grown under hGH-GFP expression inducing conditions. Error bars depict standard error of the mean (n = 4).

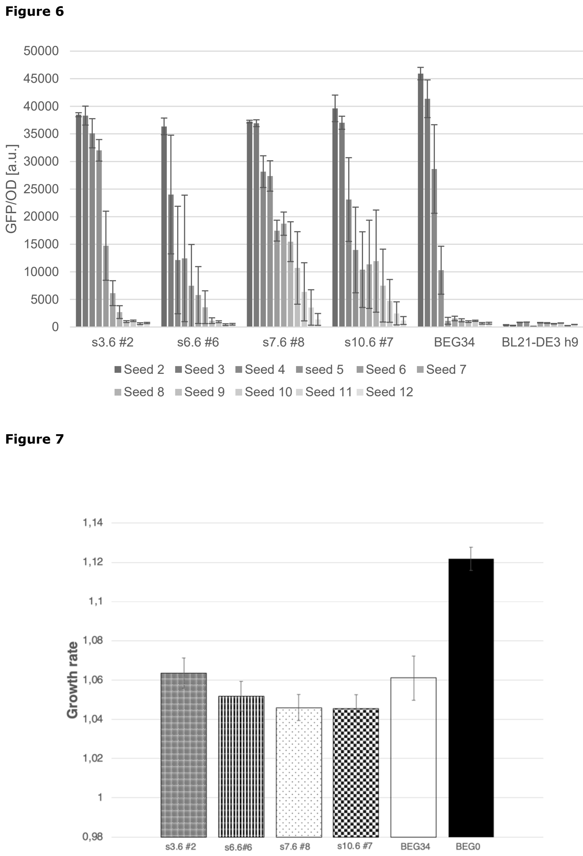

**Figure 6:** Histogram of GFP/OD values (Y-axis) measured after serial culture transfers (seed 1 to 12) of cultures of the hGH-GFP producing strains: s3.6#2 ($p_{ibpA}$), s6.6#6 ($p_{grpE}$), s7.6#8 ($p_{ycjX}$), and s10.6#7 ($p_{ybbN}$) comprising the respective burden-sensing promoters and one of four variant RBS coding sequences, driving expression of the essential gene, *folP-glmM*. Cultures of non-burden addicted *E. coli* strain BEG34 and the parent strain *E. coli* BL21(DE3) are included as controls. Measurements are performed on cultures grown under hGH-GFP expression inducing conditions. Error bars depict standard error of the mean (n = 3).

**Figure 7:** Histogram of OD/min as a measure of the growth rate of burden-addicted hGH-GFP producing strains s3.6#2, s6.6#6, s7.6#8 and s10.6#7 compared to non-burden addicted hGH-GFP producing *E. coli* strain BEG34 and the parent strain *E. coli* BL21-DE3 (BEG0). The growth rate of the respective strains is based on 7 replicate cultures measured under hGH-GFP expression inducing conditions at seed 0; error bars denote standard error of the mean (n = 7).

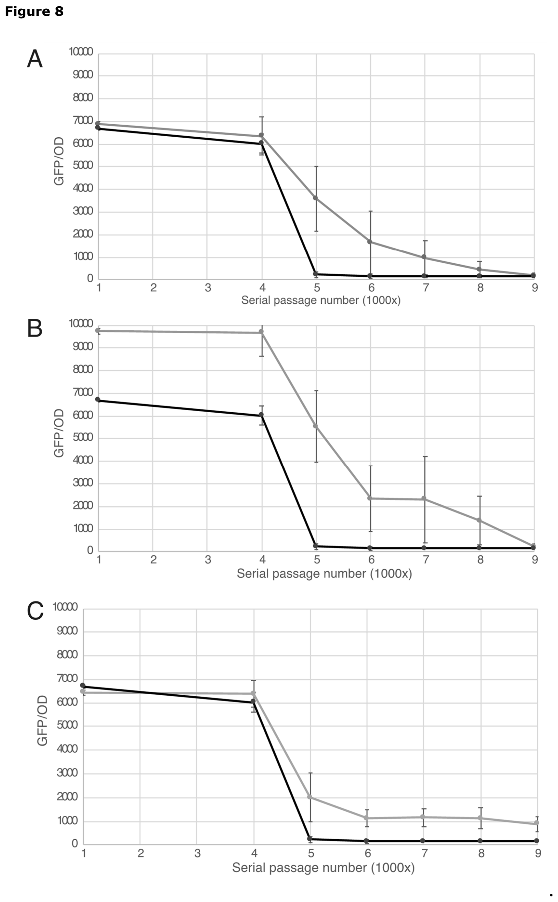

**Figure 8:** Graphs showing GFP/OD values (Y-axis) measured after serial culture transfers (seed 1, 4 to 9) of cultures of hGH-GFP producing strains, minus the values measured for an empty plasmid control BEG0-empty strain. The

strains shown in: (A) s7.6#8 ($p_{ycjX}$), (B) s13.6#2 ($p_{fsxA}$), and (C) s13.6#2evo ($p_{fsxA}$), each comprise the respective burden-sensing promoter driving expression of the essential gene, *folP-glmM* (gray line) and a non-burden addicted hGH-GFP producing E. *coli* strain BEG34 (black line). Measurements are performed on cultures grown under hGH-GFP expression inducing conditions. Error bars depict standard error of the mean (n = 5).

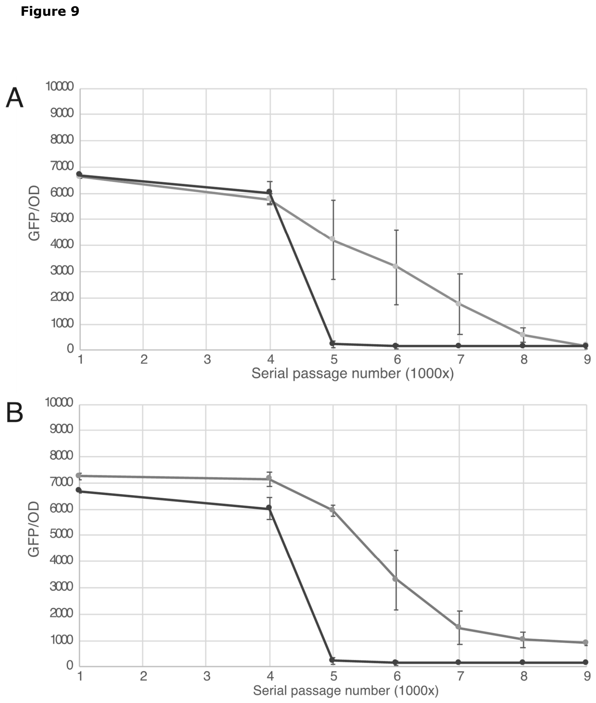

**Figure 9:** Graphs showing GFP/OD values (Y-axis) measured after serial culture transfers (seed 1, 4 to 9) of cultures of hGH-GFP producing strains, minus the values measured for an empty plasmid control BEGO-empty strain. The strains shown in: (A) s15.6.7 ($p_{rrnB}$) and (B) s16.6.6 ($p_{rrnE}$), each comprise the respective burden-sensing promoter driving expression of the essential gene, *folP-glmM* (gray line) and a non-burden addicted hGH-GFP producing *E. coli* strain BEG34 (black line). Measurements are performed on cultures grown under hGH-GFP expression inducing conditions. Error bars depict standard error of the mean (n = 5).

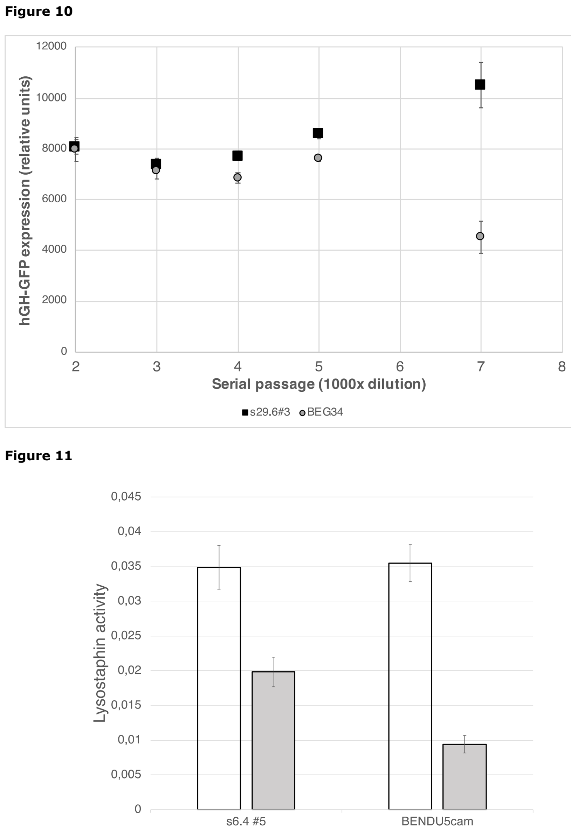

**Figure 10:** Graph showing relative hGH-GFP production (Y-axis) measured after serial culture transfers (seed 2 to 5, and 7) of cultures of a hGH-GFP producing strain, s29.6#3, minus the values measured for a parallel empty plasmid control BEGO strain.

Strain s29.6#3 comprises the osmotic stress sensing promoter $p_{poxB}$ driving expression of the essential gene, *folP-glmM* (black squares), compared to non-burden addicted hGH-GFP producing *E. coli* strain BEG34 (gray dots). Serial passage 7 was analyzed after 72 h. Measurements are performed on cultures grown under hGH-GFP expression inducing conditions. Error bars depict standard error of the mean (n = 5 for s29.6#3 and n = 8 for BEG34).

**Figure 11:** Histogram showing S. *aureus* lysis rate per minute, normalized to *E. coli* culture OD, as a measure of the secreted lysostaphin production rate of cultures of lysostaphin-producing strains s6.4#5 comprising the burden sensing promoter $p_{grpE}$ and one of four variant RBS coding sequences driving expression of the essential gene, *folP-glmM,* compared to non-burden addicted lysostaphin producing *E. coli* strain BENDU5cam. The rate was measured on cultures grown under lysostaphin expression inducing conditions after serial culture transfers (seed 1 and 7). Error bars depict standard error of the mean (n = 3).

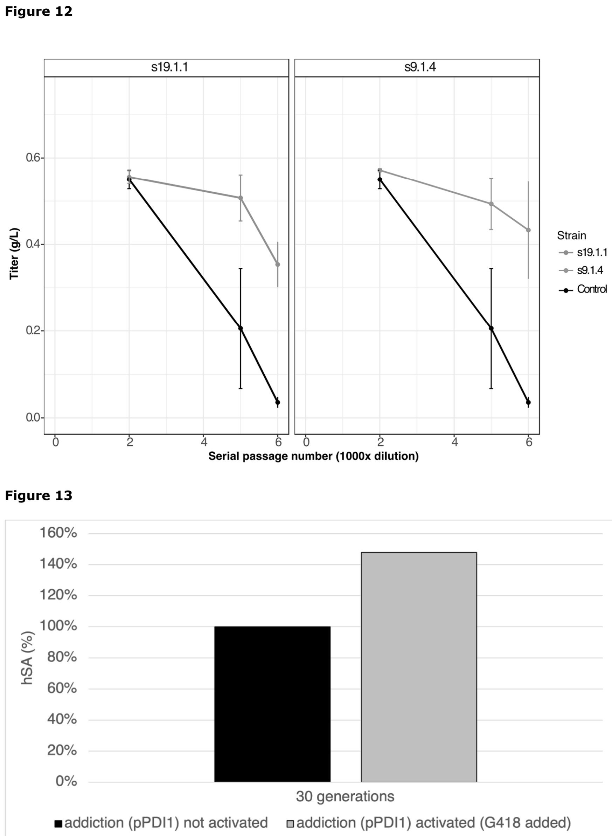

**Figure 12:** Graph showing mevalonic acid titer (g/L) synthesized by cultures of the burden-addicted mevalonic acid-producing strains s19.1#1($p_{cspD}$) and s9.1.4($p_{mutM}$), measured after serial culture transfers (seed 2, 5 and 6) compared to the parent non-burden addicted mevalonic acid-producing *E. coli* strain BL21(DE3)-pMevT. The burden sensing promoters, $p_{cspD}$ and $p_{mutM}$, are each combined with one of four variant RBS coding sequences driving expression of the essential gene, folP-glmM. Measurements are performed on cultures grown under MevT expression inducing conditions. Error bars depict standard error of the mean (n = 3).

**Figure 13:** Bar plot showing human serum albumin (hSA) production titers of cultures of *Pichia pastoris* strain EGS31 comprising a genomically-integrated copy of a cDNA version of the hSA coding *ALB1* gene under control of the *AOX1* promoter, and a *PDI1* promoter operably linked to the *kanMX* selection gene. The cultures were grown under either selecting conditions (addiction activated by 750 $\mu$g/mL G418) or non-selecting conditions (addiction not activated). The hSA titer of the 'addiction not activated' culture is set to 100%, and the titer of the 'addiction activated culture is shown as a percent thereof.

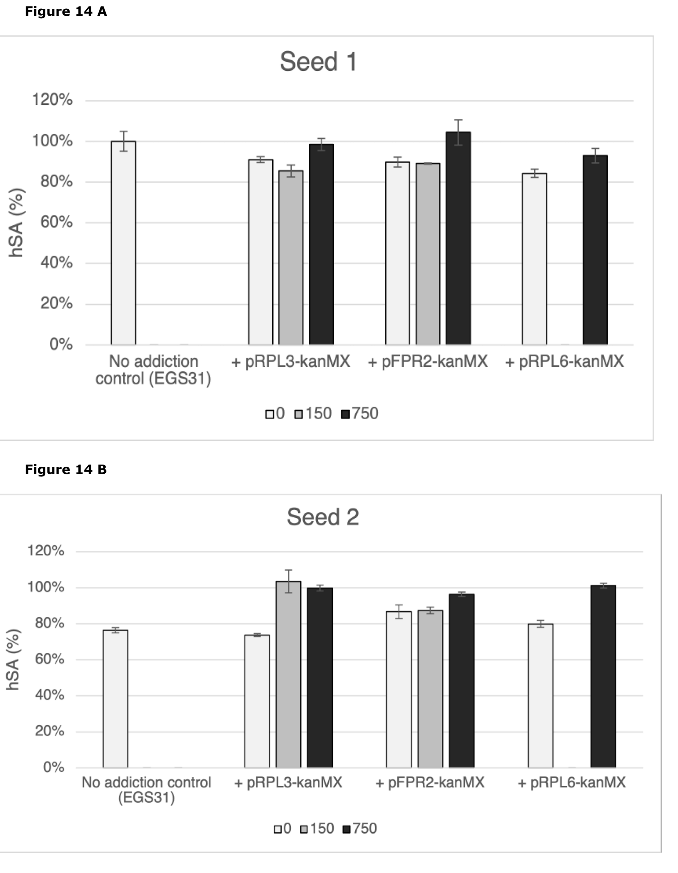

**Figure 14 A and B:** Bar plot showing human serum albumin (hSA) production titers of cultures of burden-addicted versions of the *Pichia pastoris* strain EGS31 each comprising a genomically-integrated copy of a cDNA version of the hSA coding ALB1 gene under control of the *AOX1* promoter and a *kanMX* selection gene operably fused to a burden responsive promoter *FPR2,* or *RPL3* or *RPL6A.* The cultures were grown under either non-selecting conditions (addiction not activated) or under selecting conditions (addiction activated with either 150 or 750 $\mu$g/mL G418) for a period of about 30 cell divisions ([A] Seed 1), or after a further 10 cell divisions ([B] Seed 2). Average hSA titers are shown in percent of the titer in the seed 1 'EGS31' culture. Error bars depict standard error of the mean (n = 4).

**Abbreviations, terms and definitions:**

**Burden** refers to general and/or product-specific metabolic toxicity, metabolic burden, and/or metabolic depletion; which a microbial cell genetically engineered to synthesize a product, experiences during production of the product under production conditions, and which results in a fitness cost that can be quantified by measuring the percent reduction in the maximum exponential growth rate of the cell (along the growth curve) during production of the product under production conditions as compared to a parent microbial cell incapable of said production when grown under comparable production conditions.

**Burden-sensing promoter** refers to a promoter that is induced by said 'burden', and when induced, said burden-sensing promoter can upregulate the expression of a gene to which it is operably linked in a microbial production cell, when cultured under production conditions as compared to a mutant derivative of said microbial production cell that synthesizes essentially none, or at least 50 % less, of a product of the production cell. Such mutant derivative includes a non-productive escape mutant isolated following long-term cultivation of a population of cells derived from said production cell. Non-limiting examples of burden-sensing promoters include promoters of the E. *coli* genes *htpG, ibpA, clpB, yccV, grpE, ycjX, IdhA, mutM, ybbN, prlC, groES, fxsA, htpX, rrnB, rrnE, cspD, katE, xthA, uspE, gadB, ahpC, katG, grxA, oxyS, poxB, trxC* (see Table 2 for details) and their homolog versions in other gram-positive and gram-negative bacteria; and for S. *cerevisiae* non-limiting examples of burden-sensing promoters include promoter of the genes KAR2, *PDI1, SAA1, FPR2, RPL3, RPL6A, RPL28, OGG1, RAD51, RAD54* (see Table 2 for details). Burden-sensing promoters may also be hybrids, scrambled or truncated versions of such natural promoters as long as such promoters still maintain the response to burden.

**Burden-addicted microbial cell** refers to a microbial cell engineered to comprise a genetic circuit comprising said 'burden-sensing promoter' operably linked to an essential gene of the cell.

**Burden-addicted microbial production cell** refers to microbial cell genetically engineered to synthesize a desired product, said cell being further genetically engineered to comprise a genetic circuit comprising said 'burden-sensing promoter' operably linked to an 'essential gene' of the cell. The desired product may be a protein encoded by a nucleic acid molecule in said engineered microbial cell, or a product of a heterologous pathway encoded by one or more nucleic acid molecules to be expressed by the cell.

**Desired products** of the invention include - but are not limited to - products that, when synthesized by the genetically engineered microbial production cell, convey a fitness cost (burden) upon the production cell. Non-limiting examples include organic acids, terpenoids, isoprenoids, polyketides, alcohols, sugars, vitamins, aldehydes, carboxylic acids, fatty acids, amino acids, peptides, enzymes, therapeutic proteins and precursors thereof, such as human growth hormone, insulin, glucagon-like peptide-1, monoclonal and polyclonal antibodies, single-fragment antibodies.

**Essential gene** (or essential gene operon) refers to gene(s) in the microbial production cell which, if down-regulated, lead to reduction in the growth rate of the microbial production cell under production conditions. Preferably an essential gene is essential for growth irrespective of the nutrient composition of these production conditions, whereby sufficient expression of such essential genes to support cell growth would not be dependent on the presence or absence of specific inhibitors or nutrients provided under the production conditions . Non-limiting examples of essential genes during standard laboratory conditions include *folP-glmM, glmM, murI, asd, thyA, rpoD, nusG, rpsU, accD, degS, fldA, ftsN, hflB, lolA, mraY, mreD, murA, murB, murF, nadD, rplV* and *rpsG*, and *for Bacillus licheniformis* or *Bacillus subtilis: glmM, ylaN, infA,* and *dapA,* and for S. *cerevisiae* non-limiting examples include URA3, *FOL1, MED7, RRP40, NOP8, PGI1* and *NEP1.* For example, suitable genes encode enzymes responsible for synthesizing cell wall constituents. Further, an essential gene used in the context of this invention preferably neither encodes a desired product to be expressed by the production strain; nor encodes a protein that facilitates synthesis of a desired product or intermediate thereof in a heterologous pathway to be expressed by the production strain.

**Conditional essential gene** is one that allows the burden addiction system in the microbial production cell to be "activated" by culturing the cells under the condition of essentiality such as added antibiotic or removed nutrient.

**Essential gene basal expression level** is the level of transcription of an 'essential gene' (as defined above) that is operably linked to a 'burden-sensing promoter' in a burden-addicted microbial production cell, when the 'burden-sensing promoter' is not induced. The burden sensing promoter to which the essential gene is operably linked is heterologous with respect to said essential gene, in the sense that the promoter is not the native promoter of said essential gene (even though the promoter may be present in the same genome), and is thus not found operably linked to said essential gene in nature. Said basal (i.e. un-induced) level expression of an essential gene is a level sufficient to support growth of a cell under production conditions (or during an exponential growth phase) at a level equal to or less than 10, 20, 50, 90 or 95% of the growth rate of a corresponding cell wherein said essential gene is operably linked to its native promoter.

**Fitness cost:** is quantified by comparing the maximum exponential growth rate of a non-burden addicted microbial production cell (i.e. lacking a burden-sensing promoter operably linked to the essential gene), but comprising and expressing one or more genes encoding the product or encoding a metabolic pathway for biosynthesis of the product, relative to the maximum exponential growth rate of a parent microbial cell either devoid or incapable of expressing gene(s) encoding the product or metabolic pathway for biosynthesis of the product, from which the microbial production cell was derived, when grown under comparable production conditions.

**Production conditions** refers to specific cultivation conditions to be used during production and can relate to medium and/or other culture conditions (e.g. temperature, pH, stirring, aeration, etc) for production of a desired product by the microbial production cell.

**Ribosomal binding site** (RBS), translation initiation region, or translational strength element refers to the genetic region of the 5' untranslated region that control the translation strength of a particular messenger RNA.

**Detailed description of the invention:**

**I. A burden-addicted microbial production cell**

A first aspect of the present invention provides a microbial production cell genetically engineered to synthesize a product, wherein said cell further comprises:

    a. an essential gene operably linked to a burden-sensing promoter,

wherein said promoter is heterologous with respect to said essential gene; wherein production of the product confers a burden and/or fitness cost on said cell, and wherein expression of said essential gene is up-regulated when said burden-sensing promoter is induced by said burden and/or fitness cost relative to a basal level expression of said essential gene when said burden-sensing promoter is not induced.

According to one embodiment, the microbial production cell of the invention comprises:

b. one or more genes encoding the product or encoding a metabolic pathway for synthesis of the product, wherein optionally said one or more genes are operably linked to a constitutive or inducible promoter.

According to one embodiment, said burden and/or fitness cost on said microbial production cell of the invention, is quantified by comparing the maximum exponential growth rate of the microbial production cell comprising one or more genes encoding the product or encoding a metabolic pathway for synthesis of the product, but lacking a burden-sensing promoter operably linked to the essential gene, relative to the maximum exponential growth rate of a parent microbial cell lacking or incapable of expressing one or more genes encoding the product or encoding a metabolic pathway for synthesis of the product, and from which said microbial production cell was derived, where the respective cells are cultured under essentially identical production conditions. The burden and/or fitness cost on said microbial production cell preferably corresponds to a percent reduction in the quantified maximum exponential growth rate selected from among $\geq 5$ %, $\geq 10$%, $\geq 15$%, $\geq 20$%, $\geq 25$%, $\geq 35$% and $\geq 45$ %.

The burden-addicted microbial production cell according to the invention may be any prokaryotic or eukaryotic microorganism such as a bacterium, yeast, and filamentous fungus.

In one embodiment, the microbial production cell of the invention is a prokaryote. A non-exhaustive list of suitable bacteria is given as follows: a species belonging to the genus selected from among *Escherichia, Lactobacillus, Lactococcus, Corynebacterium, Bacillus, Acetobacter, Acinetobacter, Pseudomonas; Proprionibacterium, Bacteroides* and *Bifidobacterium.*

In another embodiment, the microbial production cell of the invention is a eukaryote, such as a yeast or fungus. In certain embodiments, the eukaryote can be a member of the genus *Saccharomyces, Komagataella* or *Aspergillus.*

A non-exhaustive list of suitable yeasts is given as follows: a yeast belonging to the genus of *Saccharomyces,* e.g. S. *cerevisiae, S. kluyveri, S. bayanus, S. exiguus, S. sevazzi, S. uvarum;* a yeast belonging to the genus *Kluyveromyces,* e.g. *K. lactis K. marxianus* var. *marxianus, K. thermotolerans;* a yeast belonging to the genus *Candida,* e.g. C. *utilis C. tropicalis, C. albicans, C. lipolytica, C. versatilis;* a yeast belonging to the genus *Pichia,* e.g. *P. stipidis, P. pastoris, P. sorbitophila,* or other yeast genera, e.g. *Cryptococcus, Debaromyces, Hansenula, Yarrowia, Zygosaccharomyces* or *Schizosaccharomyces.*

A non-exhaustive list of suitable filamentous fungi is given as follows: a filamentous fungus belonging to the genus of *Penicillium, Rhizopus, Fusarium, Fusidium, Gibberella, Mucor, Mortierella, Trichoderma Thermomyces, Streptomyces* and *Aspergillus.* More specifically, the filamentous fungus may be selected from *Fusarium oxysporum, A. niger, A. awamori, A. oryzae,* and *A. nidulans.*

The product(s) synthesized by the microbial production cell of the invention is one that incurs a burden on the cell and fitness cost reflected in a reduction in growth rate, such product(s) including amino acids, organic acids, terpenoids, isoprenoids, polyketides, alcohols, sugars, vitamins, aldehydes, carboxylic acids, fatty acids, peptides, enzymes, therapeutic proteins and their precursors such as human growth hormone, insulin, glucagon-like peptide-1, monoclonal and polyclonal antibodies, single-fragment antibodies. In one embodiment the product is not a native product of the microbial production cell of the invention, and thus not produced by a parent cell from which the microbial production cell was derived. In one embodiment, the one or more genes encoding the product or encoding a metabolic pathway for synthesis of the product are heterologous with respect to the microbial production cell of the invention; where said one or more genes may be transgenes.

**II. Features of the microbial production cell of the invention**

II.i *Fitness cost - production burden*

Heterologous expression of a desired product or pathways leading to a desired product in a microbial cell imposes a burden on the cell. Burden can also result from overproduction of a native product, e.g. in cells of microbial strains selected following mutagenesis. The burden may be described as a fitness cost which results in slower growth of the cell (Example 1; figure 1). This may be due to general and/or product-specific metabolic toxicities, increased use and/or depletion of potentially finite cell resources and metabolites, which a microbial cell experiences during the biosynthesis of a product of the invention under production conditions. Particularly during industrial scale fermentation, a combination of fitness cost and slower growth places a selective pressure on the population of cells in the fermenter, which over time leads to a decline in product yield due to an accumulation of non-productive cells resulting from genetic mutations or non-genetic variation (example 2, BEG34 in figure 6).

In the present invention, the presence and expression of one or more genes encoding a product or encoding a metabolic pathway for biosynthesis of a product in a microbial production cell confers a fitness cost (burden).

The fitness cost of producing a product on a microbial production cell is quantified by comparing the growth rate of a non-burdened addicted microbial production cell (i.e. lacking burden-sensing promoter operably linked to the essential gene), but comprising and expressing one or more genes encoding the product or encoding a metabolic pathway for biosynthesis of the product, relative to the growth rate of a parent microbial cell devoid of gene(s) encoding the product or metabolic pathway for biosynthesis of the product, from which the microbial production cell was derived. The burden and/or fitness cost on said microbial production cell preferably corresponds to a percent reduction in the quantified growth rate selected from among ≥ 5 %, ≥10%, ≥15%, ≥20%, ≥25%, ≥35% and ≥45%, more preferably at least 5%.

Preferably quantification of the relative growth rates is performed on the respective microbial cells by measuring their maximum exponential growth rate when cultured under essentially identical conditions, these conditions being chosen to closely mimic those in which eventual large-scale fermentation is to take place.

II.ii *Essential gene*

A microbial production cell of the invention comprises an essential gene encoding a protein whose expression is required for cell growth and/or survival.

Preferably, the essential gene and its expression do not indirectly cause a fall in production of the desired product by the production cell. Further, essential genes used in the context of this invention do not encode or lead to synthesis of a desired product or intermediate of a heterologous pathway to be expressed in the microbial production cell.

The essential gene is preferably a non-conditional essential gene, such that expression of the gene is essential for cell growth and/or survival irrespective of the composition of the growth medium or conditions in which the cell is cultured.

In one embodiment of the invention, when the production cell is a prokaryote, such as *E. coli,* the non-conditional essential gene is selected from *folP-glmM, glmM, murI, asd, thyA, usA, rpoD, nusG, rpsU, accD, degS, fldA, ftsN, hflB, lolA, mraY, mreD, murA, murB, murF; nadD, rplV* and *rpsG.*

In a further embodiment of the invention, when the production cell is a prokaryote, such as a *Bacillus* strain, the non-conditional essential gene is selected from *glmM, ylaN, infA,* and *dapA.*

In one embodiment of the invention, the essential gene is a conditionally essential gene leading to the synthesis of a product required for auxotrophic growth, or a protein product required for resistance to growth inhibitors such as an antibiotic, specific toxin, protoxin, or the like.

In one embodiment of the invention, when the production cell is a eukaryote, the essential gene when non-conditional is selected from *FOL1, MED7, RRP40, NOP8, PGI1, NEP1;* and when conditional is selected from *URA3, LEU2, TRP1, HIS3.*

When the essential gene is conditionally essential then the composition of the production cell's growth medium/-growth conditions must be adjusted, such as using growth medium lacking specific nutrients or supplemented with growth inhibitors.

| **Table 1** Non-exhaustive list of *E. coli, S. cerevisiae* and *Bacillus* essential genes | | |
|---|---|---|
| Gene *** | Gene encoded product(s) | Ecocyc/ Biocyc accession ID** |
| **E. coli** | | |
| folP-glmM (SEQ ID NO.: 1) | Operon of genes encoding dihydropteroate synthase and phosphoglucosamine mutase | EG50011 |
| glmM (SEQ ID NO.: 3) | phosphoglucosamine mutase | EG11553 |
| murI (SEQ ID NO.: 5) | glutamate racemase | EG11204 |
| asd (SEQ ID NO.: 7) | aspartate-semialdehyde dehydrogenase | EG10088 |
| thyA (SEQ ID NO.: 9) | thymidylate synthase | EG11002 |

(continued)

| Gene *** | Gene encoded product(s) | Ecocyc/ Biocyc accession ID** |
|---|---|---|
| **Table 1** Non-exhaustive list of *E. coli, S. cerevisiae* and *Bacillus* essential genes | | |
| *E. coli* | | |
| rpoD (SEQ ID NO.: 11) | RNA polymerase, sigma70 | EG10896 |
| nusG (SEQ ID NO.: 13) | transcription termination factor NusG | EG10667 |
| rpsU (SEQ ID NO.: 15) | 30S ribosomal subunit protein S21 | EG10920 |
| accD (SEQ ID NO.: 17) | acetyl-CoA carboxyltransferase subunit β | EG10217 |
| *degS* (SEQ ID NO.: 19) | serine endoprotease | EG11652 |
| *fldA* (SEQ ID NO.: 21) | flavodoxin 1 | EG10318 |
| *ftsN* (SEQ ID NO.: 23) | cell division protein FtsN | EG11529 |
| *hflB* | ATP-dependent zinc metalloprotease FtsH | EG11506 |
| (SEQ ID NO.: 25) | | |
| *lolA* (SEQ ID NO.: 27) | outer membrane lipoprotein carrier protein | G6465 |
| *mraY* (SEQ ID NO.: 29) | phospho-N-acetylmuramoyl-pentapeptide-transferase | EG10604 |
| *mreD* (SEQ ID NO.: 31) | cell shape determining protein MreD | EG10610 |
| *murA* (SEQ ID NO.: 33) | UDP-N-acetylglucosamine 1-carboxyvinyltransferase | EG11358 |
| *murB* (SEQ ID NO.: 35) | UDP-N-acetylenolpyruvoylglucosamine reductase | EG11205 |
| *murF* (SEQ ID NO.: 37) | D-alanyl-D-alanine-adding enzyme | EG10622 |
| *nadD* (SEQ ID NO.: 39) | nicotinate-mononucleotide adenylyltransferase | G6350 |
| *rplV* (SEQ ID NO.: 41) | 50S ribosomal subunit protein L22 | EG10882 |
| *rpsG* (SEQ ID NO.: 43) | 30S ribosomal subunit protein S7 | EG10906 |
| *S. cerevisiae* | | SGD ID* |
| *FOL1* (SEQ ID NO.: 45) | dihydroneopterin aldolase | SGD:S000005200 |
| *MED7* (SEQ ID NO.: 47) | mediator complex subunit MED7 | SGD:S000005495 |
| *RRP40* (SEQ ID NO.: 49) | exosome non-catalytic core subunit RRP40 | SGD: S000005502 |
| *NOP8* (SEQ ID NO.: 51) | Nucleolar protein required for 60S ribosomal subunit biogenesis | SGD: S000005504 |
| *PGI1* (SEQ ID NO.: 53) | glucose-6-phosphate isomerase | SGD: S000000400 |
| *NEP1* (SEQ ID NO.: 55) | 18S rRNA pseudouridine methyltransferase | SGD:S000004176 |
| *URA3* (SEQ ID NO.: 57) | orotidine-5'-phosphate decarboxylase | SGD:S000000747 |
| *LEU2* (SEQ ID NO.: 59) | 3-isopropylmalate dehydrogenase | SGD: S000000523 |
| *TRP1* (SEQ ID NO.: 61) | phosphoribosylanthranilate isomerase | SGD:S000002414 |
| *HIS3* (SEQ ID NO.: 63) | imidazoleglycerol-phosphate dehydratase | SGD:S000005728 |
| *Bacillus* | | BL lotus tag**** |
| *glmM* (SEQ ID NO.; 147) | Phosphoglucosamine mutase | BL02703 |
| *ylaN* (SEQ ID NO.: 149) | Protein controlling cell shape | BL02974 |
| *infA* (SEQ ID NO.: 151) | Translation initiation factor | BL01028 |

(continued)

| Bacillus | | BL lotus tag**** |
|---|---|---|
| dapA (SEQ ID NO.: 153) | Dihydrodipicolinate synthase | BL01208 |

**** BL: accessed through https://www.genome.jp/kegg-bin/show_organism?org=bli
*** An essential gene according to the invention is one encoding a protein that has at least 70%, 80%, 90%, 95% or even 100% amino acid homology with the protein encoded by the respective essential gene listed in the Table.
** BioCyc/EcoCyc Database Collection is a large online collection of Pathway/Genome Databases - accessed through https://biocyc.org/
* SGD is the "Saccharomyces Genome Database" - accessed through https://yeastgenome.org/

The essentiality of a gene can be determined by creating cells in which the respective gene is knocked-out, where a non-conditional gene knockout will result in a loss of cell viability, or failure to grow, irrespective of growth conditions; while a conditional gene knockout will result in a failure to grow or loss of cell viability depending on the composition medium/conditions of cultivation.

A suitable essential gene can be identified by performing a test assay that swaps the native promoter of the assayed essential gene for an inducible promoter, such as the L-arabinose inducible $p_{BAD}$ (in *E. coli*),)), xylose-inducible $p_{XYL}$ (in *Bacillus subtilis* or *Bacillus licheniformis*) or galactose inducible $p_{GAL}$ (in S. *cerevisiae* or *Pichia Pastoris*), or plac (in *E. coli* or *Lactobacillus*). In such an essential gene assay, an integration DNA construct comprising the inducible promoter is chromosomally integrated into a host microbial cell using standard methods. In the case of prokaryotic organisms, the integration DNA preferably harbors an RBS catalog (e.g., Table 3) to direct translation of the essential gene at different rates to account for different baseline expression levels of different promoters and different baseline required expression levels required of essential genes to support growth at the corresponding wildtype specific growth rate. After targeted integration of the integration DNA, integrant cells are plated on plates supplemented with permissive conditions (temperature or concentration of inducers: >0.9 % L-arabinose, > 0.8% xylose, >1% IPTG, >2% galactose). Cells are subsequently tested for their synthetic addiction to the inducer condition in standard growth curve assays, and suitable essential genes can be identified as genes for which inducer-dependent growth rates can be identified. Inducer-dependent growth can be observed as a reduced growth rate of > 5 percent in absence of inducing conditions. Suitable essential genes are characterized by conferring less than < 5 % reduction in exponential-phase specific growth rate during optimal inducer conditions in production-relevant cultivation medium.

Methods for identification of an essential gene are detailed in "Gene Essentiality - Method and Protocols" January 2015 [DOI 10.1007/978-1-4939-2398-4] which enables the skilled person to determine whether a gene is essential for microbial cell growth under specific growth conditions; including methods for mapping and identifying essential genes of *Campylobacter jejuni; Streptococcus sanguinis; Porphyromonas gingivalis; Escherichia coli; Leptospirosis; Mycobacterium tuberculosis; Pseudomonas aeruginosa;* and *Candida albicans;* primarily by screening transposon tagged libraries. Additionally, a variety of "computational tools" described therein enable the skilled person to directly predict and identify genes encoding essential proteins in a microbial genome, facilitated by the widespread availability of whole genome microbial sequences; and the structural features of the many known essential genes. Accordingly the skilled person is provided with both databases of essential genes; and various freely accessible on-line tools and algorithms for successfully and reproducibly identifying large numbers of essential genes in the genome of microbial cells without undue burden. It should further be noted that 82% of the essential genes in *Saccharomyces cerevisiae* encode essential proteins that are similar to a protein in another organism [Giaever G et al., 2002]; demonstrating that most essential genes, either known or identified in one micro-organism (e.g. yeast) will have a counterpart in other microorganisms - and could thus be identified in a microorganism of choice by a simple BLAST search.

II.iii *Burden-sensing promoter*

The microbial production cell of the invention comprises a burden-sensing promoter operably linked to the essential gene encoding a protein required for cell growth and/or survival, as described above. A burden-sensing promoter is one that is induced in a microbial production cell that is subject to a burden and/or fitness cost due to its production or over-production of a product. Typically, induction of the burden-sensing promoter will occur when a microbial cell's production of a product results in a reduction in the cell's growth rate that, in turn, is attributable to the fitness cost of production. Importantly, the burden-sensing promoter, according to the invention, is induced by the "burden status" in the production cell resulting from production of a product, and not by the product *per se.* Since the use of the burden-sensing promoter and essential gene to enhance productivity of a microbial production cell is largely independent of the product it is engineered to make, the burden-addiction conferred by the invention supports a wide range of microbial production cell applications.

Since the essential gene is operably linked to the burden-sensing promoter, its level of transcription and expression is only increased above a basal level when the burden-sensing promoter is induced. A suitable burden-sensing promoter is one that, when un-induced, permits a basal level of expression of the operably linked essential gene that is significantly limiting for, or prevents cell growth and/or survival. Hence, cells comprising burden-sensing promoter in the un-induced state, will grow significantly slower that cells where the essential gene is operably linked to its native promoter (example 1, figure 2). By contrast, microbial production cells comprising the burden-sensing promoter linked to an essential gene exhibit a significantly increased growth rate when the cells synthesize the desired product as compared to cells not producing the product (example 1, figure 3). Furthermore, microbial production cells of the invention, despite the inherent burden of producing a product, can surprisingly achieve an initial growth rate that is indistinguishable from the growth rate of parent microbial cells, devoid of gene(s) encoding the product or metabolic pathway for biosynthesis of the product, and from which the microbial production cell was derived (example 1, figure 4). Hence under production conditions, a microbial production cell of the invention will have a selective advantage over an escape variant of the production cell that ceases to synthesize the product, and thereby depress the escape rate.

In one embodiment, the burden-sensing promoter may be a native promoter, or a modified native promoter, with respect to the microbial production cell, while being heterologous with respect to the essential gene.

In one embodiment, the promoter contains native TF/sigma factor binding sites. Suitable burden-sensing promoters are those of the *E. coli* σ32 regulon since they are generally upregulated in response to over-expression of proteins and their intracellular aggregation. A non-exhaustive list of *E. coli* σ32 promoters is included herein in Table 2, while additional suitable promoters are identified by Nonaka et al, 2006.

In one embodiment, the burden-sensing promoter is a σ factor regulated promoter. In a preferred embodiment, the burden-sensing promoter is activated via the σ32 regulon, and can be selected from among promoters of the *E. coli* genes *htpG, ibpA, clpB, yccV, grpE, ycjX, IdhA, mutM, ybbN, prlC, groES, fxsA,* and *htpX.* For *Bacillus,* such suitable burden-sensing promoters can be selected e.g. among HrcA regulated promoters including the promoter of *groES.*

In one embodiment, the burden-sensing promoter is the promoter of the *mutM* gene. *mutM* encodes a functionally conserved DNA-glycosylase responsible for initiating repair of one of the most common oxidative stress induced DNA damage, i.e. oxidation of guanine to 7,8-dihydro-8-oxoguanine (8-oxoG) (Jain et al, 2007).

Burden-sensing promoters also include promoters related to a cell's carbon nutrient status and growth phase. Accordingly, in one embodiment, the burden-sensing promoter is the promoter of the toxin gene, *cspD,* which is activated by reduced growth rates and carbon starvation (Uppal et al., 2014; Yamanaka and Inouye, 1997) and hence predicted to be differentially up-regulated in cells subject to burden or fitness cost due to production of metabolites.

In one embodiment, the burden-sensing promoter is a ribosomal RNA promoter, in particular a promoter of *rrnB* and *rrnE* genes, that respond to the nutritional supply, are induced by the Fis protein, and are inhibited by ppGpp alarmone. *rrn* promoter activity is induced in cells with high protein production, as seen in their early exponential growth phase (Nonaka et al, 2006).

In one embodiment, the burden-sensing promoter is one responsive to oxidative stress associated with metabolic burden and microbial heterologous production (Dragosits & Mattanovich, 2013). Oxidative stress response promoters include those promoters regulated by OxyR, which activates several genes associated with amelioration and protection of the cell in response to oxidative damage; and promoters of genes belonging to rpoS (σS) that are upregulated during overexpression of proteins primarily during stationary growth phase.

| Table 2 Non-exhaustive list of burden-sensing promoters | | | |
|---|---|---|---|
| No.* | Promoter | Encoded protein of *E. coli* or *S. cerevisiae* gene regulated by the promoter | Ecocyc/ Biocyc accession ID** |
| 2 | p(*htpG*) (SEQ ID NO.: 65) | Heat shock protein G (HtpG), a member of the Hsp90 family, is a molecular chaperone that aids the refolding of protein aggregates in the cell. *htpG* transcription is activated via the σ32 regulon. (Yeast homolog: Hsp90) | EG10461-MONOMER |
| 3 | p(*ibpA*) (SEQ ID NO.: 66) | Small heat shock proteins LbpA and LbpB are chaperone proteins associated with heterologous protein expression and inclusion body formation that aids refolding of aggregated protein. *ibpA* transcription is activated via the σ32 regulon. | EG11534 |
| 4 | p(*clpB*) (SEQ ID NO.: 67) | ClpB is a caseinolytic protease catalyzing protein disaggregation. *clpB* transcription is activated via the σ32 and/or σ70 regulon. (Yeast homolog: Hsp104). | EG10157 |

(continued)

| No.* | Promoter | Encoded protein of *E. coli* or *S. cerevisiae* gene regulated by the promoter | Ecocyc/ Biocyc accession ID** |
|---|---|---|---|
| | | **Table 2** Non-exhaustive list of burden-sensing promoters | |
| 5 | p(*yccV*) (SEQ ID NO.: 68) | YccV also known as HspQ participates in degradation of denatured protein during heat shock. *yccV* transcription is activated via the σ32 regulon. | G6500 |
| 6 | p(*grpE*) (SEQ ID NO.: 69) | GrpE is a nucleotide exchange factor in the DnaK-DnaJ-GrpE chaperone system. *grpE* promoter is activated via the σ32 regulon. | EG10416 |
| 7 | p(*ycjX*) (SEQ ID NO.: 70) | *ycjX* is activated via the σ32 regulon. | G6659 |
| 8 | p(*ldhA*) (SEQ ID NO.: 71) | D-lactate dehydrogenase and the *ldhA* promoter is activated via the σ32 regulon. | G592 |
| 9 | p(*mutM*) (SEQ ID NO.: 72) | DNA-formamidopyrimidine glycosylase is a component of the base excision repair pathway repairing certain types of DNA lesions. *mutM* promoter is activated via the σ32 regulon, but is inhibited by DksA-ppGpp binding under amino acid starvation. | EG10329 |
| 10 | p(*ybbN*) (SEQ ID NO.: 73) | YbbN also known as CnoX is a chaperedoxin. The *ybbN* promoter is activated via the σ32 regulon. | G6268 |
| 11 | p(*prlC*) (SEQ ID NO.: 74) | PrlC also known as oligopeptidase A; and belongs to the σ32 regulon. | EG11441 |
| 12 | p(*groES*) (SEQ ID NO.: 75) | GroES is a chaperonin, and belongs to the σ32 and σ70 regulon. | EG10600 |
| 13 | p(*fxsA*) (SEQ ID NO.: 76) | FxsA is a polytopic membrane protein belonging to the σ32 regulon. | G7832 |
| 14 | p(*htpX*) (SEQ ID NO.: 77) | HtpX is a zinc-dependent endoprotease and heat shock protein that belongs to the σ32 regulon. | EG10462 |
| 15 | p(*rrnB*) (SEQ ID NO.: 78) | 16S rRNA gene, whose promoter responds to nutritional supply, and is induced in cells with high protein production. | EG30085 |
| 16 | p(*rrnE*) (SEQ ID NO.: 79) | 16S rRNA gene, whose promoter responds to nutritional supply, and is induced in cells with high protein production. | EG30088 |
| 19 | p(*cspD*) (SEQ ID NO.: 80) | CspD is a toxin. The *cspD* promoter responds inversely with growth rate. Transcription of *cspD* is activated by cyclic-AMP-CRP, H-NS and ppGpp. | EG11111 |
| 21 | p(*katE*) (SEQ ID NO.: 81) | Monofunctional catalase (HPII) responsible for decomposing the toxic hydrogen peroxide into water and oxygen in the cell. *katE* transcription is dependent on $\sigma^S$ and activated by Fur-$Fe^{2+}$ and ppGpp. | EG10509 |

(continued)

| No.* | Promoter | Encoded protein of *E. coli* or *S. cerevisiae* gene regulated by the promoter | Ecocyc/ Biocyc accession ID** |
|---|---|---|---|
| | | **Table 2** Non-exhaustive list of burden-sensing promoters | |
| 22 | *p(xthA)* (SEQ ID NO.: 82) | Multifunctional endonuclease, Exodeoxyribonuclease III, that e.g. removes DNA damaged due to hydrogen peroxide. *xthA* belongs to the $\sigma^{32}$ regulon. | EG11073 |
| 23 | *p(uspE)* (SEQ ID NO.: 83) | Universal stress protein E; which is induced in stationary phase as a result of various stress conditions such as oxidative stress. *uspE* transcription is activated by GadX and ppGpp. | EG11246 |
| 24 | *p(gadB)* (SEQ ID NO.: 84) | Glutamate decarboxylase B helps the cell maintain a near-neutral intracellular pH upon exposure of acidic conditions by converting glutamate into gamma-aminobutyrate, accepting one proton in the reaction. *gadB* is a part of the the $\sigma^{70}$ and $\sigma^{S}$ regulon, and is activated by AdiY, GadE, GadX, $P^{asp56}$- RcsB and ppGpp. | EG11490 |
| 25 | *p(ahpC)* (SEQ ID NO.: 85) | Alkyl hydroperoxide reductase C plays a role in protecting cells from oxidative stress by catalyzing the reduction of endogenously synthesizes hydroperoxide. *ahpC* is part of the $\sigma^{70}$ and transcription is activated by OxyR. | EG11384 |
| 26 | *p(katG)* (SEQ ID NO.: 86) | Bifunctional hydroperoxidase I (HPI) that acts both as a catalase and a peroxidase. | EG10511 |
| 27 | *p(grxA)* (SEQ ID NO.: 87) | Glutaredoxin is mainly responsible for catalyzing the reduction of disulfides. Transcription initiation of *grxA* is activated by OxyR. | EG10417 |
| 28 | *p(oxyS)* (SEQ ID NO.: 88) | *oxyS* is a small RNA gene activated by oxidative stress via OxyR. | EG31116 |
| 29 | *p(poxB)* (SEQ ID NO.: 89) | Pyruvate oxidase that is upregulated by oxidative and osmotic stress via OxyR, but also by Cra, MarA, fnr, ppGpp and $\sigma^{s}$ | EG10754 |
| 30 | *p(trxC)* (SEQ ID NO.: 90) | Thioredoxin that is induces during oxidative stress via OxyR | EG11887 |
| | *p(KAR2)* (SEQ ID NO.: 91) | HAC-upregulated promoter comprising an unfolded proteins response element, positively responding to high production | |
| | *p(PDI1)* (SEQ ID NO.: 92) | HAC-upregulated promoter comprising an unfolded proteins response element, positively responding to high production | |
| | *p(SAA1)* (SEQ ID NO.: 93) | HAC-upregulated promoter comprising an unfolded proteins response element, positively responding to high production | |
| | *p(FPR2)* (SEQ ID NO.: 94) | HAC-upregulated promoter comprising an unfolded proteins response element | |
| | *p(RPL3)* (SEQ ID NO.: 95) | RNA polymerase I upregulated promoter, positively responding to high production | |

(continued)

| No.* | Promoter | Encoded protein of *E. coli* or *S. cerevisiae* gene regulated by the promoter | Ecocyc/ Biocyc accession ID** |
|------|----------|-------------------------------------------------------------------------------|-------------------------------|
| | p(RPL6A) (SEQ ID NO.: 96) | RNA polymerase I upregulated promoter, positively responding to high production | |
| | p(RPL28) (SEQ ID NO.: 97) | RNA polymerase I upregulated promoter, positively responding to high production | |
| | p(OGG1) (SEQ ID NO.: 98) | DNA damage response, resulting from heterologous expression broadly induces transcription of the DNA repair system. | |
| | p(RAD51) (SEQ ID NO.: 99) | DNA damage response, resulting from heterologous expression broadly induces transcription of the DNA repair system. | |
| | p(RAD54) (SEQ ID NO.: 100) | DNA damage response, resulting from heterologous expression broadly induces transcription of the DNA repair system. | |

*Table 2 Non-exhaustive list of burden-sensing promoters*

* No. corresponds to the numbers assigned to the different strains tested in the Examples section of the present application.
** BioCyc/EcoCyc Database Collection is a large online collection of Pathway/Genome Databases - accessed through https://biocyc.org/

The basal expression level and specific response curve upon induction/activation of the different burden-sensing promoters (e.g. in Table 2) vary, which can be exploited when selecting a burden-sensing promoter having the best match (in terms of strength) to a specific microbial production cell and its product.

Suitable burden-sensing promoters that are induced by the burden and/or fitness cost in a microbial production cell during production can be selected from public databases known to the skilled person, for example https://ecocyc.org for *E. coli* promoters; https://bsubcyc.org for *Bacillus subtilis* promoters (which also works for *Bacillus licheniformis* promoters), their respective prokaryotic homologues; https://www.yeastgenome.org for *Saccharomyces cerevisiae* promoters, and their fungal homologues.

Suitable burden-sensing promoters can also be validated in a test assay in which a candidate burden-sensing promoter is operably linked to a gene encoding a fluorescent protein (e.g. green fluorescent protein) and integrated into a microbial production cell genetically engineered to synthesize a product, and integrated into a corresponding non-producing cell (control), which was isolated following a serial dilution cultivation experiment in production medium for between 50-150 cell generations. Burden-sensing promoters induce detectable fluorescent protein expression in producing cells compared to the non-producing cell (control) by at least 5 %, 7.5 %, 10 %, 15 %, 25 %, 60 %, 150 %, 300 %.

II.iv *Matching a burden-sensing promoter to a microbial production cell*

Microbial production cells, in response to the burden or fitness cost of producing a product, exhibit a transcriptional state characterized by the expression of certain genes, whose expression is not induced in a corresponding non-productive parent cell or a non-productive escape cell derived from a microbial production cell during production fermentation. The physiological nature of the burden or fitness cost will depend on the product synthesized by a given microbial production cell, and will be reflected in the types of genes whose expression is induced. The identity of the gene promoters induced in a cell producing a given product can be determined by techniques well-known in the art (e.g. transcriptomics, see example 7). Since many of the induced gene promoters will be related to, or correspond to those listed in Table 2, these provide a starting point for finding a matching burden-sensing promoter for a given microbial production cell. The choice of promoters can be extended by identifying 5 to 15 promoters of genes found to be specifically upregulated in the production strain of interest. As illustrated in the examples herein, the method used to match a burden-sensing promoter operably linked to an essential gene to a microbial production cell in a product-specific manner, is experimentally fast and can be conducted in simple laboratory setups.

II.v *Translation control elements*

Regulating the growth of a microbial production cell of the invention by means of essential gene expression, requires that the response threshold and curve of the burden-sensing promoter and the expression level of the essential gene are balanced; such that basal level essential gene expression supports limited or no growth, while in highly productive cells the induced burden-sensing promoter drives sufficient essential gene expression to support a significantly increased growth rate, preferably a growth rate similar to that of productive cells lacking the burden-sensing promoter of the invention or ≤5 % lower, when measured in the exponential growth phase.

One suitable approach to balancing the burden-sensing promoter's burden-response to the expression level of the essential gene is to modify the translational strength of the essential gene. In bacteria, translational strength is defined by the Shine-Dalgarno/ribosome binding site (RBS) sequence directly upstream of the start codon, while in eukaryotic cells translation initiation regions/Kozak elements can be used to modify translational strength. RBSs in *E. coli* conferring a broad range of translational strengths are provided in Table 3, while further examples can be found in the literature (e.g. Bonde et al, 2016). A skilled person in the art can balance the translational strength of the regulated essential gene by constructing four variants of the ribosomal binding site for each burden-sensing promoter (Rugbjerg et al, 2018, PNAS), and testing which variant enables a selected essential gene to effectively regulate the growth rate of a cell. Exemplary RBSs for use in *Bacillus licheniformis* or *Bacillus subtilis* are provided in Table 3.1.

| **Table 3** List of suitable RBSs in *E. coli* generally covering a broad expression range as defined in assay by expression of red fluorescent protein (Bonde et al, 2016). | |
| --- | --- |
| RBS | Log (Expression level) |
| ACTTGA | 2.80244 |
| ACTTGC | 2.73094 |
| ACTTGG | 3.14847 |
| ACTTGT | 2.77523 |

| **Table 3.1** List of suitable RBSs in *B. subtilis* or *B. licheniformis* generally covering a broad expression range. |
| --- |
| RBS |
| ACGAGA |
| ACGAGC |
| ACGAGT |
| ACGAGA |

II.vi *Engineering burden-sensing promoters and translational control element*

A native burden-sensing promoter of choice is generally encompassed by the -1 to -300 bp region (upstream) of the native regulated ORF in prokaryotic organisms and the -1 to -500 bp region in eukaryotic organisms. Core promoters that must be included and the sequence boundaries of their regulatory sites e.g. transcription factor binding sites, are common general knowledge, such as for σ32 (Nonaka et al, 2006). The translational control element/RBS can be added downstream of the promoter, to avoid alteration in the regulatory properties of the selected promoter sequence.

II.vii *Improved production levels*

A surprising advantage of the microbial production cell of the invention, in addition to having an increased initial growth rate as compared to non-productive cells, is that the cells retain significantly improved productivity during a large scale fermentation (simulated in example 2; figure 6), over many cell divisions, compared to microbial production cells lacking engineered burden-addiction. The surprising advantages conferred by burden-addiction in a microbial production cell of the invention are obtained irrespective of the type of product synthesized - as illustrated by the range of products synthesizes by microbial production cells in examples 2-5.

According to one embodiment, the microbial production cell of the invention is characterized by improved product yield following at least 20, 25, 30, 35, 40, 45, 50, 55, 60, 70, 100, 150, 250 or 400 generations of cell division from a single cell, compared to a non-burden-addicted production cell following the same generations of cell division. Product yield is measured as moles or grams of product produced per unit substrate.

In one embodiment, production levels are increased by at least 10, 25, 50, or 80% following at least 50 generations of cell division from single cell, compared to a reference non-burden-addicted production cell following the same generations of cell division.

Universal practice in the engineering of microbial cell factories has aimed at minimizing the burden known to arise due to maintenance and expression of heterologous pathways. The present invention provides a counter-intuitive solution to improving cell factory yields, since it makes microbial cell growth and survival dependent on the cell producing its product while being subject to a constant burden or fitness cost. While not wishing to be bound by theory, it is speculated that the selection pressure placed on a microbial production cell of the invention and its descendants, where only productive cells in a state of burden can grow/survive, may serve to progressively select for highly-burdened cell sub-populations during long-term culture in a starting population of initially isogenic cells.

### III. Methods for preparing and identifying cells of a burden-addicted microbial production strain

In a preferred method of performing the invention, a burden-addicted microbial production strain is prepared and identified by the following steps. To obtain the best working relation between burden sensing promoter and essential gene, it may be relevant to prepare and test different candidate burden-sensing promoters with different RBSs for regulating a selected essential gene.

*III.i Identification of burden-sensing promoter candidate(s) in a specific production strain*

Candidate burden-sensing promoters listed in Table 2 may be tested. Alternatively, unique, positively differentiating gene transcripts detected in a specific production strain of interest may be identified; whose respective promoters provide a source of candidate burden sensing promoters. Preferably such differentiating gene transcripts are identified by comparing the transcript profile of a microbial production strain (during production) with an isolated genetic or non-genetic escape mutant variant(s) derived from the corresponding production strain, where such escape mutant variant is characterized by at most a 50 % lower production rate. Positively differentiating gene transcripts can be identified by use of transcriptomics; for example by RNA sequencing of the transcribed RNA extracted from productive microbial production cell(s) compared to the non/low-producing escape mutant variant. Identified promoters can be tested in combination with a given essential gene in a microbial production cell.

A method for identifying one or more burden-sensing promoters is illustrated in example 7; where after said promoter(s) is operably linked to an essential gene in a chosen microbial production cell for testing.

*III.ii Introduction of burden-sensing promoter in growth-regulating processes*

One or more promoter(s) selected from the list of general candidate burden-sensing promoters (Table 2) or from identified specific burden-sensing promoter(s) (see IIIi), are tested by operably linking it to an essential gene in the microbial production cell. Different translation strengths of the essential gene can be simultaneously tested by providing alternative RBS sequences for the cognate essential gene; as well as testing different essential genes (as describe in section IIIi). The essential gene may be native or heterologous with respect to the microbial production cell.

When the essential gene is a native gene, its cognate native promoter may be disrupted (i.e. made non-functional) by e.g. mutation or deletion events, and the burden-sensing promoter (that is heterologous with respect to the essential gene) is then operably linked to the native essential gene by targeted introduction. In another embodiment, the native essential gene promoter is replaced with the burden-sensing promoter by targeted introduction.

Suitable methods for targeted introduction of a genetic sequence in microorganisms include recombineering in bacteria, e.g. Lambda Red recombineering in *E. coli;* while homologous recombination can be used in yeasts and filamentous fungi. Both concepts can optionally be used in combination with CRISPR to increase gene replacement efficiency.

*III.iii Screening for balanced burden-sensing and growth-regulation*

Microbial production cell clones comprising an inserted burden-sensing promoter operatively linked to an essential gene are then screened to identify clones where the burden-sensing promoter regulates an essential gene in a growth-controlling manner. For example, the growth of a number of such clones (e.g. 8-96 clones) is compared with cells of a corresponding non-burden-addicted microbial production strain, under conditions were product production is controlled (e.g. using microbial production strains where the production gene is inducible).

When cell growth is measured under conditions where product production by both strains is low/absent; a suitable burden-addicted production clone is one that exhibits a significant, and preferably at least 5 %, lower growth rate than the non-burden-addicted production strain (Example 1, figure 2).

III.iv *Validation of non-perturbed central and production metabolism*

Changes in transcriptional regulation or expression of essential genes can lead to unwanted, indirect perturbation of the production genes and central carbon or nitrogen metabolism, compromising product formation and in turn reduced burden in a burden-addicted microbial production cell. In order to exclude such clones, cell growth is measured under conditions where both strains synthesize product; where a suitable burden-addicted production clone is one that exhibits a growth rate equal to or lower than the non-burden-addicted production strain (as seen in example 2, figure 7).

III.v *Screening for growth-rate stability*

Clones that fulfil the above criteria are tested for growth rate stability under production-mimicking conditions by cultivation for at least 20 generations of cell division, e.g. by serial passage and measurement of growth rates, or by taking samples from various steps in a scaled-up production process. Suitable burden-addicted microbial production cells maintain growth rates lower than the non-burden-addicted production strain over time (e.g. example 2, figure 7).

**IV. Method for producing a desired product using cells of a burden-addicted microbial production strain**

A second aspect of the present invention concerns a method for producing a desired product comprising the steps of:

a. providing a microbial production cell genetically engineered to synthesize a product, wherein said cell further comprises an essential gene operably linked to a burden-sensing promoter, and wherein said promoter is heterologous with respect to said essential gene,

b. introducing the genetically modified microbial cell into a cultivation medium comprising substrate for production of said product,

c. recovering said product synthesizes by said culture,

wherein synthesis of the product confers a burden and/or fitness cost on said cell, and wherein expression of said essential gene is up-regulated relative to basal level expression of said essential gene when said burden-sensing promoter is induced by said burden and/or fitness cost; and wherein a lack of said product synthesis in said burden-addicted production strain or progeny cell thereof reduces growth rate of said cell.

**IV. Use of cells of a burden-addicted microbial production strain for producing a desired product**

A third aspect of the present invention concerns the use of a burden-addicted microbial production cell of the invention for producing a desired product, wherein a lack of product synthesis in said a burden-addicted production strain or progeny cell thereof reduces growth rate of said strain.

**NUMBERED EMBODIMENTS**

Numbered embodiment 1: A microbial production cell genetically engineered to synthesize a product, said microbial cell further comprising:

a. an essential gene operably linked to a burden-sensing promoter,

wherein said promoter is heterologous with respect to said essential gene;
wherein synthesis of the product confers a burden on said cell, and wherein expression of said essential gene is up-regulated when said burden-sensing promoter is induced by said burden relative to a basal level expression of said essential gene when said burden-sensing promoter is not induced.

Numbered embodiment 2: The microbial production cell according to numbered embodiment 1, wherein said cell comprises

b. one or more genes encoding the product or encoding a metabolic pathway for synthesis of the product, wherein said one or more genes are operably linked to a promoter.

Numbered embodiment 3: The microbial production cell according to numbered embodiment 1 or 2, wherein said burden conferred by synthesis of said product, when said essential gene in the cell is operably linked to its native promoter, has a fitness cost measured as a percent reduction in the exponential phase growth rate of the microbial production cell selected from among $\geq 5\%$, $\geq 10\%$, $\geq 15\%$, $\geq 20\%$, $\geq 25\%$, $\geq 35\%$ and $\geq 45\%$ relative to a corresponding non-producing microbial cell.

Numbered embodiment 4: The microbial production cell according to any one of numbered embodiments 1-3, wherein the cell is:

i) a bacterium belonging to a genus selected from among *Escherichia, Lactobacillus, Lactococcus, Corynebacterium, Bacillus, Acetobacter, Acinetobacter, Pseudomonas; Proprionibacterium, Bacteroides,* and *Bifidobacterium;* or
ii) a yeast belonging to a genus selected from among *Saccharomyces, Kluyveromyces, Candida, Pichia, Komagataella, Cryptococcus, Debaromyces, Hansenula, Yarrowia, Zygosaccharomyces* and *Schizosaccharomyces;* or
iii) a filamentous fungus selected from among *Penicillium, Rhizopus, Fusarium, Fusidium, Gibberella, Mucor, Mortierella, Trichoderma Thermomyces, Streptomyces* and *Aspergillus.*

Numbered embodiment 5: The microbial production cell according to any one of numbered embodiments 1-4, wherein the essential gene is a non-conditional essential gene.

Numbered embodiment 6: The microbial production cell according to any one of numbered embodiments 1-5, wherein the cell is a bacterium and the essential gene is an *E. coli* gene or operon selected from among *folP-glmM, glmM, murI, asd, thyA, usA, rpoD, nusG, rpsU, accD, degS, fldA, ftsN, hflB, lolA, mraY, mreD, murA, murB, murF, nadD, rplV* and *rpsG* or a homologue thereof.

Numbered embodiment 7: The microbial production cell according to any one of numbered embodiments 1-6, wherein the essential gene is operably linked to a synthetic RBS whose sequence is selected to modify the translational strength of the essential gene independent of induction of said burden-sensing promoter.

Numbered embodiment 8: The microbial production cell according to any one of numbered embodiments 1-7, wherein the burden-sensing promoter is selected from among:

i) a $\sigma$ factor regulated promoter, such as a $\sigma^{32}$, $\sigma^{B}$ or $\sigma^{S}$ factor regulated promoter,
ii) a ribosomal RNA promoter,
iii) an HAC1-upregulated promoter comprising a UPR element, and
iv) a DNA damage-sensing promoter.

Numbered embodiment 9: The microbial production cell according to any one of numbered embodiments 1-8, wherein the cell is characterized by an increased product yield after at least 25, 30, 35, 40, 45, 50, 60, 70, 80, 90 or 100 generations of cell division from a single cell, as compared to a parent microbial production cell lacking said essential gene operably linked to a burden-sensing promoter.

Numbered embodiment 10: The microbial production cell according to any one of numbered embodiments 1-9, wherein the cell is characterized by an increased product yield of at least 10, 25, 50, or 80% following at least 50 generations of cell division from a single cell as compared to a parent microbial production cell lacking said essential gene operably linked to a burden-sensing promoter.

Numbered embodiment 11: A method of product biosynthesis comprising the steps of:

i) providing a microbial production cell according to any one of numbered embodiments 1-10,
ii) introducing the cell into a cultivation medium comprising substrate for production of said product,
iii) recovering said product.

Numbered embodiment 12: A method of product biosynthesis according to numbered embodiment 11, wherein the product is selected from among an: amino acid, organic acid, terpenoid, isoprenoid, polyketide, alcohol, sugar, vitamin, aldehyde, carboxylic acid, fatty acid, peptide, enzyme, therapeutic protein and precursor thereof, human growth hormone, insulin, glucagon-like peptide-1, monoclonal and polyclonal antibody, and single-fragment antibody.

Numbered embodiment 13: Use of an essential gene operably linked to a burden-sensing promoter to enhance product yield of a cultured population of microbial production cells arising from a single cell, wherein said promoter is heterologous with respect to said essential gene, wherein production of the product confers a burden on said cell, and wherein expression of said essential gene is up-regulated when said burden-sensing promoter is induced by said burden relative to a basal level expression of said essential gene when said burden-sensing promoter is not induced.

Numbered embodiment 14: Use of a microbial production cell according to any one of numbered embodiments 1-9 for producing a biosynthetic product.

Numbered embodiment 15: Use of a microbial production cell according to numbered embodiment 14, wherein the product is selected from among an: amino acid, organic acid, terpenoid, isoprenoid, polyketide, alcohol, sugar, vitamin, aldehyde, carboxylic acid, fatty acid, peptide, enzyme, therapeutic protein and precursor thereof, human growth hormone, insulin, glucagon-like peptide-1, monoclonal and polyclonal antibody, and single-fragment antibody.

## EXAMPLES

**Example 1: Engineering burden-addicted *E. coli* strains producing recombinant human growth hormone**

**1.1 *Maintenance and expression of heterologous production genes by a cell factory constitutes an unnatural burden on its cells***

The growth rate of parent *E. coli* BL21(DE3) cells was compared with an engineered derivative transformed with the plasmid, pEG34, comprising a gene encoding recombinant human growth hormone (hGH) fused to Green Fluorescent Protein (GFP).

1.1.1 Materials and Methods

Cells of the host strain *E. coli* BL21(DE3) (E. *coli* Genetic Stock Center at Yale University), were made electrocompetent and transformed by standard electroporation methods (1800 V, 25 $\mu$F, 200 Ohms, 1 mm cuvette width) with the plasmid pEG34, and plated on LB agar plates containing chloramphenicol.

| Table 4 Plasmids | | |
|---|---|---|
| Plasmid | Genotype | Expressed proteins |
| pEG34 [SEQ ID No.: 101] | hGH-GFP fusion camR gene* [SEQ ID No.: 103] | hGH fused to GFP [SEQ ID No.: 102] Chloramphenicol resistance protein [SEQ ID No.: 104] |
| pEG0 [SEQ ID No.: 105] | *camR* gene* [SEQ ID No.: 103] | Chloramphenicol resistance protein [SEQ ID No.: 104] |
| pENDU5CAM [SEQ ID No.: 106] | *lss* gene *camR* gene* [SEQ ID No.: 103] | Lysostaphin [SEQ ID No.: 107] Chloramphenicol resistance protein [SEQ ID No.: 104] |
| pMevT [SEQ ID No.: 108] | *mevT* operon | Acetoacetyl-CoA synthase [SEQ ID No.: 109] Hydroxymethylglutaryl-CoA synthase [SEQ ID No.: 110] Hydroxymethylglutaryl-CoA reductase |
| | *camR* gene* [SEQ ID No.: 103] | [SEQ ID No.:111 ] Chloramphenicol resistance protein [SEQ ID No.: 104] |
| pSIM5-tet [Koskiniemi et al 2011] | | |
| *camR* gene [SEQ ID No: 103] encoding chloramphenicol resistance protein [SEQ ID No: 104] is in pEG34 [SEQ ID No: 101] located at nucleotide positions 3768-4427 on the complementary strand; is in pEG0 [SEQ ID No: 105] located at nucleotide positions 2339-2995 on the complementary strand; is in pENDU5CAM [SEQ ID No: 106] located at nucleotide positions 1617-2276 on the complementary strand; and is in pMevT [SEQ ID No: 108] located at nucleotide positions 6585-7244 on the complementary strand. | | |

Pre-cultures of single colony transformants were cultured in 96-well microtiter plates on 200 $\mu$L 2xYT medium (16

g/L tryptone, 5 g/L NaCl, 10 g/L yeast extract) containing 500 μM IPTG and 30 mg/L chloramphenicol at 37 °C with fast horizontal shaking in an ELx808 plate reader (Biotek) with reads at OD630 every ten minutes. OD630 values were background-subtracted using the OD630 value of the first read.

1.1.2 Results

*E. coli* cells comprising the hGH-GFP expression plasmid, pEG34, grow slower than cells of the parent *E. coli* host from which is was derived (figure 1); illustrating the burden or load placed on cells as a result of allocating cellular resources to maintenance and expression of a synthetic construct.

### 1.2 *Engineering a burden-addicted cell factory*

Burden-addiction strains of the parent *E. coli* host were genetically engineered to incorporate a genetic circuit designed to confer a selective fitness advantage on productive cells in a cell factory. Specifically, a burden-sensing promoter ($p_{mutM}$, $p_{yccV}$ or $p_{ycjX}$), was substituted for the native promoter of the essential gene operon *folP-glmM* in the *E. coli* BL21(DE3) chromosome. The genetic circuit further included an RBS, between the promoter and essential gene, where four different RBSs (Table 3) were tested for modulating the expression level of the essential gene. The growth rate of strains comprising the genetic circuit was compared with the parent host strain *E. coli* BL21(DE3).

1.2.1 Materials and Methods

*Burden-sensing promoters:* The promoters, $p_{mutM}$, $p_{yccV}$ or $p_{ycjX}$, were generated by PCR amplifying a 0.3 kb region immediately upstream of the respective gene (see Table 3) using primers specified in Table 5 and genomic DNA derived from lysed *E. coli* BL21(DE3) cells as template. The PCR mix: 10 μl MQ water, 2 μl forward primer (10μM), 2 μl revers primer (10μM), 1μl DNA template, 15 μl Phusion U MasterMix (Thermo Scientific). The PCR reaction protocol: 95°C for 180 sec (1x); 95 °C for 20 sec, 68-58°C (touchdown) for 30 sec, 72°C for 60 sec (35x); 72°C for 300 sec (1x); leave at 15°C.

| **Table 5 Primers** | | | |
|---|---|---|---|
| Promoter of *E. coli* gene | Primer name | Primer sequence (5'-3')* | SEQ ID No.: |
| yccV | yccV for | ATTCATATGUGGTTATAAAGACATCAACATGC | 112 |
| | yccV rev | ATCCCCATTACGTGAGGATGGCTAAGGTCCAG TGAAGTACCCTGGGCAAAGAGTTTCATacctt**N CAAGT**AGTCACCTCCCGGGAAATCT | 113 |
| ycjX | ycjX for | ATTCATATGUTTGGCTGATACTGTAATTCTTC | 114 |
| | ycjX rev | ATCCCCATTACGTGAGGATGGCTAAGGTCCAG TGAAGTACCCTGGGCAAAGAGTTTCATacctt**N CAAGT**TGCTGTCCTGTGCTGCTCTG | 115 |
| mutM | mutM for | ATTCATATGUGGCGAAAAGTACTAAGTACTTA | 116 |
| | mutM rev | ATCCCCATTACGTGAGGATGGCTAAGGTCCAG TGAAGTACCCTGGGCAAAGAGTTTCATacctt**N CAAGT**AGCATCTCCAGGAATGAACA | 117 |
| | P492 | TGCAACTCTTCTTCAACGC | 118 |
| | P493 | AAGAACCAGGCGCTTCTAA | 119 |
| | P525 | ATTGATGACCTGATGGCAC | 120 |
| | P526 | GATCCAGACTTCGAAGCG | 121 |
| * RBS complementary coding sequences are in bold font; wherein N is either A, G, T or C. The RBS is encoded on the opposite strand of the oligo sequence shown. | | | |

USER cloning was used to generate integration sequences comprising the amplified promoter region fused to a linear 1.5 kb DNA fragment containing a *kanR* gene for selection of correct recombineering products and a 221 bp targeting sequence identical to 221 bp directly upstream of the *folP* gene. The amplified promoter region and kanR-folP fragment were fused by mixing them in equimolar amounts and adding 1μl 10x T4 ligation buffer (Thermo Scientific) and 0.75 μl USER enzyme (New England Biolabs), in a total 10μl reaction volume. The USER reaction was placed at 37°C for 30 minutes. The reaction was then placed at room temperature for 15 minutes followed by the addition of 0.75 μl T4 DNA ligase (Thermo Scientific) and incubation at room temperature for 30 minutes. An example of such integration sequence comprising promoter, KanR resistance gene, and folP targeting sequence can be found in the sequence listing SEQ ID NO. 140 (s9_pmutM_folP). The ligated product was then amplified using primers 'rev' (according to specific promoter in Table 5) and P493 (Table 5) to approximately 250 ng/μl using the PCR reaction protocol: 98°C for 180 sec (1x); 85°C for 20 sec, 72-68°C (touchdown) for 30 sec, 72°C for 60 sec (35x); 72°C for 5 min (1x); leave at 15°C. Primer overhang provided 50 bp *folP* identical targeting sequence to direct recombineering to the *folP* locus.

*Chromosomal integration of burden-addiction promoters:* promoters were integrated upstream of the *folP* gene in the genome of *E. coli* BL21(DE3) cells as follows: 100 ml of 2xYT medium containing tetracycline were inoculated with 600μl of BL21(DE3) overnight culture pre-transformed with pSIM5-tet (Koskiniemi et al, 2011). The cells were cultured at 30°C, and upon reaching OD600=0.20, the culture was transferred to a 42°C shaking bath for 15 minutes to allow for the expression of the recombineering enzymes located on pSIM5-tet. The culture was then transferred to 2x cold 50ml centrifuge tubes; centrifuged at 4000g for 10 min; the supernatant was discarded; and the remaining cell pellets were washed with 20 ml ice-cold 10% glycerol. The partially re-suspended cells were then centrifuged at 4000g for 6 min; the supernatant was discarded; and the cell pellets washed with 20 ml ice-cold 10% glycerol. The partially re-suspended cells were centrifuged at 4000g for 6 min; each cell pellet was carefully re-suspended in 495 μl ice-cold 10% glycerol and pooled. 90 μl of re-suspended cells were added to electroporation cuvettes each containing 1 μl (>250 ng) of one of the burden-sensing promoter integration sequences. The cells were electroporated with the following settings: 1800 V, 25 μF, 200 Ohms, 1 mm cuvette width. 900 ml 2xYT-media was added to the cuvette straight after electroporation and the cells were left to recover for 1.5 hour at 37°C in 1.5 ml Eppendorf tubes. The electroporated cells were incubated at room temperature overnight to allow time for recombination; and subsequently plated on LB agar plates containing 50 mg/L kanamycin and cultured at 37°C overnight to ensure curing for pSIM5-tet. Correct targeting to the essential gene locus was validated using primers P525 and P526. Generally, single kan[R] colonies were picked having a size average or smaller than the population of colonies.

Burden-addiction promoter integration in the genome of the kanR selected colonies was validated by colony PCR using Taq DNA polymerase and primers targeted to the *folP* promoter region. Additionally, the identity of the RBS in the selected colonies was determined by Sanger sequencing.

Subsequently, the growth rate of strains comprising the genetic circuit was compared with the parent host strain *E. coli* BL21(DE3) by cultivation in 200μl 2xYT supplemented with 500 μM IPTG at 37 °C with horizontal shaking.

1.2.2 Results

The growth rate of the selected burden-addicted *E. coli* strains were slower, to various degrees, compared to parent *E. coli* host from which they were derived (figure 2; showing growth of burden-addicted strains s5.0#3, s7.0#8, and s9.0#8 wherein essential genes *folP-glmM* are controlled by burden-sensing promoters $p_{yccV}$, $p_{ycjX}$, and $p_{mutM}$, respectively). Since cellular growth is dependent on the level of expression of the essential *folP-glmM* genes, it may be concluded that the level of essential gene expression driven by the respective burden-sensing promoter in a non-productive, burden-addicted strain is insufficient to support growth at the level of a wildtype parent strain.

The observed reduction in growth rate in these burden-addicted strains provides a measure of the penalty that may be exerted on a non-producing cell that evolves spontaneously during cultivation of a cell factory population comprising the burden-addiction genetic circuit. The degree of penalty is determined by the choice of the burden-sensing promoter combined with the strength of the chosen RBS. The larger the penalty - the wider the window for "negative selection" of any low- or non-producing variants (e.g. resulting from mutation in production genes) spontaneously arising during cultivation.

**1.3 Production increases growth rate of a burden-addicted cell factory**

The burden-addicted *E. coli* strains, which conferred a growth penalty for non-production (figure 2), were used as host strains for demonstrating the selective fitness advantage for burden-addicted cells that are productive.

1.3.1 Materials and methods

The slowest-growing clones of the generated burden-addicted *E. coli* strains (s9.0#8; s5.0#3; and s7.0#8) were made electrocompetent and transformed by standard electroporation methods (1800 V, 25 $\mu$F, 200 Ohms, 1 mm cuvette width) with respectively pEG34 or pEG0 (Table 4), and plated on LB agar plates containing chloramphenicol and kanamycin. Single colony transformants were cultured over-night on 2xYT medium containing chloramphenicol, and then used to inoculate 96-well microtiter plates comprising 200 $\mu$L 2xYT medium (500 $\mu$M IPTG, chloramphenicol) and cultured at 37 °C with fast horizontal shaking in an ELx808 plate reader (Biotek) with reads at OD630 every ten minutes. OD630 values were background-subtracted using the OD630 value of the first read.

*E. coli* strains were each transformed with the plasmid pEG34 or pEG0 (Table 4), and their growth rate properties were measured when the cells were induced to synthesize the recombinant protein, hGH fused to GFP.

1.3.2 Results

The burden-addicted strain, *E. coli* strain s9.0#8, comprising the burden sensing promoter $p_{mutM}$ controlling of the *folP-glmM* essential genes expression, exhibited the slowest growth of the strains tested when compared to the parent *E. coli* BL21 (DE3) strain (figure 2). This reduction of exponential growth rate is reversed in cells of the *E. coli* strain s9.0#8 transformed with the pEG34 plasmid and induced to express the recombinant protein, hGH-GFP, in contrast to cells of *E. coli* strain s9.0#8 transformed with the empty plasmid, BEG0 (figure 3). This demonstrates that the burden-sensing promoter $p_{mutM}$ in *E. coli* strain s9.0#8 is induced by the burden or load on the cells brought about by synthesis of the recombinant protein hGH-GFP. This, in turn, leads to up-regulated expression of the *folP-glmM* genes and enhanced exponential growth.

The synthesis of hGH-GFP in each of the burden-addicted strains, *E. coli* strain s5.0#3, s7.0#8, and s9.0#8 harboring the pEG34 plasmid, not only led to a reversal of the reduction of exponential growth rate; but additionally the growth rate of these strains was indistinguishable from growth of the parent *E. coli* BL21 (DE3) (figure 4).

*In summary,* this example illustrates that the burden-addiction genetic circuit can be used to confer a selective fitness advantage on those cells of a cell factory whose synthesis of proteins or metabolites is sufficiently high to constitute a burden or load; and that this burden or load is detectable by a burden-sensing promoter operably linked to an essential gene in the cells. By contrast, non-productive variant cells (e.g. resulting from production gene mutations), that spontaneously appear during cultivation of the burden-addicted cell factory, will be subject to a negative selection pressure, since their growth will be slowed to a rate supported by the basal expression of the essential gene. A reduced growth rate is, of itself, sufficient to slow the increase in frequency of such variant cells in the cell factory and thereby delay the decline in cell factory productivity over time.

**Example 2: Burden addicted *E. coli* strains producing human growth hormone show enhanced long-term production stability**

Burden-sensing promoters are shown to be promoters that can sense and be activated by a burden-induced state in a cell resulting from the cells synthesis of a recombinant protein, hGH-GFP. Once activated, burden-sensing promoters are shown to elevate expression of an essential gene, *folP-glmM,* to a level sufficient to confer a selective growth advantage on a cell when compared to a non-productive cell. In order to maximize the dynamic range of essential gene expression in response to its cognate burden sensing promoter, the burden-sensing promoter is randomly combined with variant RBS coding sequences (Table 3) conferring different translational strengths. Promoters having burden-sensing properties suitable for use in a burden-addiction genetic circuit are shown to include heat-shock-, DNA damage-, oxidative stress response-promoters and rRNA promoters, as illustrated by the following engineered production strains cultured under simulated large-scale production conditions.

Firstly, a number of clones harboring random variant RBS coding sequences were selected for each of the burden-sensing promoters, and their hGH-GFP synthesis was followed over many cell divisions in order to demonstrate their relative ability to elevate/preserve recombinant hGH-GFP synthesis over time.

2.1 Materials and Methods

*Burden-sensing promoters:* The promoters, $p_{yccV}$, $p_{ycjX}$, $p_{ibpA}$, $p_{grpE}$, $p_{ldhA}$ and $p_{ybbN}$, and their respective integration sequences were generated by PCR and USER cloning as described for Example 1.2.1, using the primers specified in Table 6; while the ligated products were amplified using primers'rev' (according to specific promoter in Table 6) and P493 (Table 5).

| Table 6 Primers | | | |
|---|---|---|---|
| Promoter of *E. coli* gene | Primer name | Primer sequence (5'-3')* | SEQ ID No.: |
| ibpA | ibpA fwd | ATCCCCATTACGTGAGGATGGCTAAGGTCCAGT GAAGTACCCTGGGCAAAGAGTTTCATaccttNCA AGTAATCAATAGCTCCTGAAATC | 122 |
| | ibpA rev | ATTCATATGTTTGGCCTGATGAGTTATAGCG | 123 |
| grpE | grpE fwd | ATTCATATGUTCTCCGCGAGCGTGCCAGTTTT | 124 |
| | grpE rev | ATCCCCATTACGTGAGGATGGCTAAGGTCCAGT GAAGTACCCTGGGCAAAGAGTTTCATaccttNCA AGTGAATTTCTCCGCGTTTTTTT | 125 |
| ldhA | ldhA fwd | ATTCATATGUGGGAACCCACAGCCCGAGCGTC | 126 |
| | ldhA rev | ATCCCCATTACGTGAGGATGGCTAAGGTCCAGT GAAGTACCCTGGGCAAAGAGTTTCATaccttNCA AGTAAGACTTTCTCCAGTGATGT | 127 |
| ybbN | ybbN fwd | ATTCATATGUCCCATTCGTACTCGCTTCACCGAT CCCCATTACGTGAGGATGGCTAAGGTCCAGTGA AGTACCCTGGGCAAAGAGTTTCATaccttNCAAG TGGAGTCGCTCTCTGTTGTCG | 128 |
| | ybbN rev | ATCCCCATTACGTGAGGATGGCTAAGGTCCAGT GAAGTACCCTGGGCAAAGAGTTTCATaccttNCA AGTGGAGTCGCTCTCTGTTGTCG | 129 |
| fxsA | fxsA fwd | ATTCATATGUTAAACACACAGAATATATGTGG | 130 |
| | fxsA rev | ATCCCCATTACGTGAGGATGGCTAAGGTCCAGT GAAGTACCCTGGGCAAAGAGTTTCATaccttNCA AGTAGGTTTCTCCTGTAATAGCA | 131 |
| rrnB | rrnB fwd | ATTCATATGUGCAAACACGCCGCCGGGTCAG | 132 |
| | rrnB rev | ATCCCCATTACGTGAGGATGGCTAAGGTCCAGT GAAGTACCCTGGGCAAAGAGTTTCATaccttNCA AGTAAAAGTTTGACGCTCAAAGA | 133 |
| rrnE | rrnE fwd | ATTCATATGUAATGCGCCTCCGTTGAGACGACA AC | 134 |
| | rrnE rev | ATCCCCATTACGTGAGGATGGCTAAGGTCCAGT GAAGTACCCTGGGCAAAGAGTTTCATaccttNCA AGTAAAAGTTTGATGCTCAAAGA | 135 |

(continued)

| Table 6 Primers | | | |
|---|---|---|---|
| Promoter of *E. coli* gene | Primer name | Primer sequence (5'-3')* | SEQ ID No.: |
| poxB | poxB fwd | ATTCATATGUTAGGTTGTCGCTGCCTGCCGTG | 136 |
| | poxB rev | ATCCCCATTACGTGAGGATGGCTAAGGTCCAGT GAAGTACCCTGGGCAAAGAGTTTCATacctt**NCA AGT**GGTTCTCCATCTCCTGAATG | 137 |
| * RBS complementary coding sequences are in bold font; wherein N is either A, G, T or C. The RBS is encoded on the opposite strand of the oligo sequence shown. | | | |

*Chromosomal integration of burden-addiction promoters:* each of the promoters was integrated upstream of the *folP* gene in the genome of *E. coli* strain BEG34 (corresponding to *E. coli* BL21(DE3) harboring plasmid pEG34), as follows. Cells of the *E. coli* strain BEG34, pre-transformed with the recombineering plasmid pSIM5-tet, were prepared and transformed by electroporation with each of the promoter integration sequences, as described in example 1.2.1. Following the described steps of recombineering and curing of pSIM5-tet, eight colonies were picked from each plate, corresponding to eight clones with the same promoter integration but having a random variant RBS sequence (see Table 6). Each clone was then transferred to a well of a 96-well plate containing 200 µl 2xYT supplemented with chloramphenicol for maintenance of the plasmid, pEG34. 2 µl of each cultured clone was used to validate promoter integration via colony PCR as described in example 1.2.1; prior to freezing the 96-well plate.

*Short term hGH-GFP production screening assay:* The frozen 96-well plate was thawed and a pin replicator used to transfer cells into a new 96-well plate containing 200 µL 2xYT supplemented with chloramphenicol. This plate was sealed with Breathe-Easy sealing membrane (Sigma-Aldrich) and placed in a SynergyH1 plate reader (Biotek) overnight at 37°C and 754 rpm linear shaking for 20 hours; and the OD (600nm) and GFP fluorescence (ex/em 485nm/528nm) of each well was measured every 10 minutes over a 20 hour period. The plate was then placed in a regular tabletop plate shaker at room temperature for 4 hours followed by the transfer of 2 µL culture from each well to a new 96-well plate containing 200 µL 2xYT supplemented with chloramphenicol and 0.5mM IPTG. The new 96-well plate was likewise sealed with Breathe-Easy sealing membrane (Sigma-Aldrich) and placed in a SynergyH1 plate reader overnight at 37°C and 754 rpm linear shaking for 20 hours; and the OD and GFP fluorescence of each well was measured at 600nm and ex/em 485nm/528nm every 10 minutes for 20 hours. The following day 2 µL culture was transferred to a new 96-well plate with 200 µl 2xYT supplemented with chloramphenicol and 0.5mM IPTG and the process was repeated every day for 6 days in total.

*Long term hGH-GFP production assay (figure 6):* A single selected colony from each burden-addicted hGH-GFP producing strain was used to inoculate a 15 mL Greiner culture tube containing 4 mL 2xYT supplemented with chloramphenicol and 0.5mM IPTG. The cultures were grown for 23 hours at 37°C on a 250 rpm shaking table. 2 µL of each culture were then seeded in 2000x dilution (corresponding to approx. 11 generations of cell division) into a new Greiner culture tube under identical conditions, where these method steps were repeated for a total of 12 seeds. After each passage, 200 uL samples of the culture were taken to determine hGH-GFP synthesis by measuring OD600 and GFP fluorescence (ex/em 485nm/528nm) in a SynergyH1 plate reader (Biotek).

*Long term hGH-GFP production assay (figure 8):* A single selected colony from each burden-addicted hGH-GFP producing strain was used to inoculate a 24 deep-well plate carrying 1.8 mL 2xYT supplemented with chloramphenicol and 0.5mM IPTG. The cultures were grown for 23 hours at 30°C on a 200 rpm shaking table. 2 µL of each culture were then seeded in 1000x dilution (corresponding to approx. 10 generations of cell division) into a new deep well plate under identical conditions, where these method steps were repeated for a total of 9 seeds. After each passage, 200 uL samples of the culture were taken to determine hGH-GFP synthesis by measuring OD600 and GFP fluorescence (ex/em 485nm/528nm) in a SynergyH1 plate reader (Biotek).

*Growth rate measurements of chosen strains:* Selected high-hGH producing strains, s3.6#2, s6.6#6, s7.6#8 and s10.6#7, were streaked on LB agar plates containing chloramphenicol and kanamycin. BEG34 and BEG0 strains were streaked on LB agar plates containing chloramphenicol. 7 colonies from each plate were used to inoculate a 96-well plate containing 200 µl 2xYT supplemented with chloramphenicol; which were sealed with Breathe-Easy sealing membrane (Sigma-Aldrich). Their growth was measured in a SynergyH1 plate reader (Biotek) overnight at 37°C and 754 rpm linear shaking. 2 µl samples from each well were transferred to wells in a new 96-well plate containing 200 µl 2xYT supplemented with chloramphenicol and 0.5mM IPTG. The 96-well plate was sealed with breathe-easy film and incubated for 20 hours at 37°C and 754 rpm linear shaking and growth (OD600nm) was measured every 10 minutes using a Synergy H1 plate reader.

*Strain catalogue:*

BEG34 = *E. coli* BL21(DE3) carrying pEG34;

BEG0 = *E. coli* BL21(DE3) carrying empty plasmid pEG0

sX.Z#Y = E. coli BL21(DE3) carrying pEG34, having wt folP promoter swapped for burden addiction-promoter X, wherein X refers to the promoter IDs in Table 2. The number Y following # indicates the selected clone from the pool of four RBS variants introduced by degenerate primer (Table 7). Z refers to the production genes for hGH-GFP (0 or 6), mevalonic acid (1), lysostaphin (4), respectively.

**Table 7:** Identity of the "N" nucleotide of the RBS of the respective *E. coli* strains

| Strain identifier | RBS "N" identity | Strain identifier | RBS "N" identity | Strain identifier | RBS "N" identity |
|---|---|---|---|---|---|
| s5.0 #3 | C | s16.6#6 | A | s19.6#2 | A |
| s7.0 #8 | A | s18.6#8 | T | s21.6#8 | C |
| s9.0#8 | NGS | s9.1#1 | C | s23.6#4 | T |
| s6.4#5 | C | s15.1#1 | A | s24.6#2 | T |
| s3.6#2 | A | s19.1#1 | T | s25.6#1 | C |
| s6.6 #6 | C | s14.1#5 | T | s29.6#3 | T |
| s7.6#8 | T | s15.1#5 | A | s22.6#8 | T |
| s10.6#7 | A | s19.1#7 | A | s26.6#3 | A |
| s13.6#2 | C | s15.6 #1 | A | s29.6#4 | T |
| s15.6#7 | A | s16.6#7 | T | s30.6#6 | T |

2.2 Results

*2.2.1 Short term hGH-GFP productivity screening:* The productivity of four hGH-GFP producing strains comprising burden-sensing promoters selected from a group of heat shock promoters ($p_{ibpA}$, $p_{grpE}$, $p_{ycjX}$, and $p_{ybbN}$) and having one of four RBS coding sequence variants was tested under simulated large-scale production conditions, as follows. The strains were serially passaged by 100x back-dilution every day for 6 consecutive days corresponding to ca. 6 generation per seed. By day 6 (seed 6), several strains showed an elevated hGH-GFP synthesis compared to the non-burden addicted production strain BEG34, both during seed 1 and seed 6. The strains having high short term hGH-GFP productivity relative to strain BEG34 are s3.6#2 ($p_{ibpA}$), s6.6#6 ($p_{grpE}$), s7.6#8 ($p_{ycjX}$), and s10.6#7 ($p_{ybbN}$) - see figure 5.

*2.2.2 Enhanced long term hGH-GFP productivity:* hGH-GFP productivity of burden-addicted strains, s3.6#2, s6.6#6, s7.6#8, s10.6#7, and non-burden addicted strains BEG34 and non-producing strain BEG0 was monitored under simulated large-scale fermentation by serial transfer. Following seed 2, burden-addicted strains s3.6#2, s6.6#6, s7.6#8, s10.6#7 and the non-burden addicted control strain BEG34 performed equally well in terms of hGH-GFP synthesis (figure 6. However, after an additional ca. 20 generations (seed 4), hGH-GFP productivity by the control strain, BEG34, had declined considerably; and by seed 6, the strain essentially ceased production. Surprisingly, the burden-addicted strain s7.6#8 (with $p_{ycjX}$ controlling *folP-glmM*) still retained a productivity of around 50 % of seed 2 level. Additionally, burden-addicted strains s3.6#2 (with $p_{ibpA}$ controlling *folP-glmM*), s10.6#7 (with $p_{ybbN}$ controlling *folP-glmM*), and s6.6#6 (with $p_{grpE}$ controlling *folP-glmM*) also showed significantly better long-term productivity as compared to the non-burden addicted strain BEG34.

*2.2.3 Growth rate of burden addicted strains:* The growth rates of hGH-GFP producing burden-addicted strains s3.6#2, s6.6#6, s7.6#8 and s10.6#7 is no higher than the non-burden addicted hGH-GFP production strain BEG34; and hence their improved hGH-GFP production levels over time does not to simply stem from a reduced in initial production level and thus inherently lower burden, (figure 7).

*2.2.4 Dependence of burden addiction genetic circuit on selected essential gene:* A hGH-GFP producing burden-addicted strain (s13.6#2 (fxsA)) comprising the heat shock promoter $p_{fxsA}$ controlling the essential gene *folP-glmM* was compared with a mutant derivative comprising a frame-shift *folP* abolishing folP expression, strain s13.6#2evo (fxsA), whose growth was solely dependent on glmM expression. Although the loss of folP expression led to a lower growth rate (data not shown) in complex 2xYT medium, the essential gene *glmM,* alone is shown to be sufficient to confer enhanced long-term hGH-GFP production stability in the burden-addicted strain s13.6#2evo(fxsA) at levels comparable to the strain s7.6#8 (pycjX), when compared to the non-burden addicted hGH-GFP producing strain BEG34 (figure 8).

2.2.5 *Use of ribosomal RNA promoters in the burden addiction genetic circuit:* In addition to heat-shock promoters, ribosomal RNA promoters such as $p_{rrnB}$ and $p_{rrnE}$ are shown to be capable of sensing a burden-addicted state in a cell and, in response, to control essential gene expression such as to enhance long-term productivity in a hGH-GFP production strain. As seen in figure 9, the long-term stability of hGH-GFP production in strains s15.6.7 ($p_{rrnB}$) and s16.6.6 ($p_{rrnE}$), was significantly improved when compared to a non-burden addicted hGH-GFP producing *E. coli* strain BEG34, under simulated large scale production corresponding to around 110 generations of cell division.

2.2.6 *Use of oxidative stress sensing promoters in the burden addiction genetic circuit:* Oxidative stress-sensing promoters such as $p_{poxB}$ are shown to be capable of sensing a burden-addicted state in a cell and, in response, to control essential gene expression such as to enhance long-term productivity in a hGH-GFP production strain. As seen in figure 10, both production levels and long-term stability of hGH-GFP production in strain s29.6#3 ($p_{poxB}$), was significantly increased when compared to a non-burden addicted hGH-GFP producing *E. coli* strain BEG34, under simulated large scale production of seven serial passages corresponding to approximately 90 generations and time-extended cultivation for the final seed. As seen in figure 10, production using the burden addiction genetic circuit based on $p_{poxB}$ surprisingly resulted in elevated production over time, indicative of the enrichment of non-genetically high performing variants in the population.

*In summary,* this example illustrates that burden addiction enhances long-term stability and production in *E. coli* strains engineered to synthesize human growth hormone fused to GFP by coupling transcription of essential genes *folP-glmM* to any one of a *E. coli* heat shock, oxidative stress and DNA damage responsive promoter as well as an rRNA promoter.

## Example 3: Burden addicted *E. coli* strains producing lysostaphin show enhanced long-term production stability

Lysostaphin is a 27 kDa endopeptidase that cleaves crosslinking pentaglycine bridges in the cell wall peptidoglycan of *Staphylococcus aureus* resulting in cell lysis. This antibacterial agent can be synthesizes recombinantly in *E. coli.* The burden-addiction genetic circuit comprising a promoter having burden-sensing properties is shown to enhance the production stability of such *E. coli* strains engineered to synthesize lysostaphin, as illustrated by the following burden-addicted *E. coli* strains producing lysostaphin under simulated large-scale production conditions. The productivity of the burden-addiction production strains is further shown to be optimized by selecting a strain where the burden-sensing promoter is combined with an RBS sequence (Table 6) conferring an optimized translational strength of the cognate essential gene. These advantages are exemplified by burden-addicted strain s6.4#5 ($p_{grpE}$), described below, that is shown to be more resilient to loss of production due to addiction to the burden or fitness cost resulting from lysostaphin expression.

### 3.1 Materials and Methods

*Burden-sensing promoters:* A promoter integration sequence comprising the promoter $p_{grpE}$ was generated as described for example 1.2.1, by amplifying the $p_{grpE}$ promoter by PCR, using the $p_{grpE}$-specific primers specified in Table 6.

*Chromosomal integration of burden-addiction promoters:* the *grpE* promoter was integrated upstream of the *folP-glmM* gene in the genome of *E. coli* strain BENDU5cam (corresponding to *E. coli* BL21(DE3) harboring a lysostaphin producing plasmid pENDU5cam), as follows. Cells of the *E. coli* strain BENDU5cam, pre-transformed with the recombineering plasmid pSIM5-tet, was prepared and transformed by electroporation with the $p_{grpE}$ promoter integration sequence, as described in example 1.2.1. Following the described steps of recombineering and curing of pSIM5-tet, 5 colonies were picked, corresponding to clones with the same promoter integration but having a random variant RBS sequence (see Table 6). Each clone was then transferred to a 15 mL Greiner culture tube containing 4 mL 2xYT supplemented with chloramphenicol for maintenance of the plasmid, pENDU5cam, and cultured. 2 μl of each cultured clone was used to validate promotor integration as described in example 1.2.1; prior to freezing.

*Lysostaphin production screening:* The *E. coli* strain s6.4#5($p_{grpE}$) having the highest lysostaphin-productivity was identified using a screening assay in which the three other potential combinations of RBS and $p_{grpE}$ were cultured in 2xYT supplemented with chloramphenicol in 96-well format over six serial passages of 1000x dilution (corresponding to 60 generations) as compared to the non-burden addicted production strain BENDU5cam. The strains were grown in 3mL 2xYT media containing 30 mg/L chloramphenicol in 15 mL culture tubes at 37°C for 21 hours. 150 μL of the cultures were mixed with 50 μl 50% glycerol and stored in a 96-well plate at -80°C. Additionally, 30 μL of the cultures were transferred to 3 ml fresh 2xYT media supplemented with chloramphenicol and grown for another 21 hours at identical conditions; and repeated for a total of 5 transfers where freeze-stocks were made of the overnight cultures with each transfer.

*Lysostaphin expression and detection assay:* The 96-well plate containing all freeze-stocks from the 5 transfers were thawed on ice. 20 μL from each well were transferred to a 96-well plate containing 180 μL 2xYT supplemented with

chloramphenicol, and cultured grown at 37°C in a plate reader until most wells had reached OD630=0.35; and then 10 μl 2xYT supplemented 30 mg/L chloramphenicol and 20 mM IPTG was added to each well to a final concentration of 1 mM IPTG, sufficient to induce lysostaphin gene expression. The induced cultures were grown at 37°C for 4 hours, and the plate was then centrifuged at 4000 RPM for 10 minutes. 100 μl supernatant was transferred to a new 96-well plate containing 100 μl overnight *S. aureus* culture. The *S. aureus* lysis in each well was monitored at OD630 on a plate reader at 37°C for 2 hours every 10 minutes. The S. *aureus* lysis rate was quantified by dividing the change in S. *aureus* OD by the timeframe of 60 minutes that the change occurred in. The equation can be seen below:

$$Rate_{lysis} = \frac{OD_{t_0} - OD_{t_1}}{t_1 - t_0}$$

The specific lysostaphin synthesis rate was determined by normalizing the measured rate to the final OD630 measurement of the respective production *E. coli* culture.

3.2 Results

The burden-addicted lysostaphin-producing strains having a $p_{grpE}$ promoter and one of four RBS coding sequence variants controlling expression of the essential genes, *folP-glmM,* were cultivated under simulated large-scale production conditions, achieved by serial passaging. As seen in figure 11, strain s6.4#5 ($p_{grpE}$) maintained significantly higher levels of lysostaphin production as compared to the steep reduction in production seen in the non-burden addicted lysostaphin-production BENDU5cam strain. Since the initial lysostaphin production rates for s6.4#5 ($p_{grpE}$) and BENDU5cam strains at seed 0 were the same; the smaller decline in production rates for s6.4#5 ($p_{grpE}$) is not due to an inherently lower initial burden from lysostaphin production.

*In summary:* Burden-addiction was demonstrated to enhance long-term stability and production in *E. coli* engineered to synthesize secreted lysostaphin by coupling essential genes *folP-glmM* transcription to the *E. coli* heat shock promoter $p_{grpE}$.

**Example 4: Burden addicted *E. coli* strains producing mevalonic acid show enhanced long-term production stability**

E. coli BL21(DE3) cells were engineered to synthesize mevalonic acid by introducing a plasmid pMevT expressing a heterologous three-step enzymatic pathway (Martin et al., 2003) that converts glucose to mevalonic acid via the acetyl-CoA pool. A burden-addiction genetic circuit comprising a promoter having burden-sensing properties and controlling transcription of an essential gene is shown to enhance the production stability of such mevalonic acid-producing *E. coli* strains under simulated large-scale production conditions. The productivity of the burden-addiction production strains is shown to be further optimized by selecting a strain where the burden-sensing promoter in combined with a RBS coding sequence (Table 5) conferring an optimized translational strength of the cognate essential gene. Specifically, mevalonic acid-production strains comprising the burden-addiction promoter, $p_{cspD}$ ( an oxidative stress and glucose starvation sensing promoter), or $p_{mutM}$ (heat shock/DNA damage-sensing promoter) controlling *folP-glmM* transcription, are both shown to be more resilient to loss of production than the non-burden addicted strain due to their addiction to transcriptional signals resulting from mevalonic acid production.

4.1 Materials and Methods

*Burden-sensing promoters:* Promoter integration sequences comprising the promoters $p_{mutM}$ and $p_{cspD}$ were generated as described for example 1.2.1, by amplifying the $p_{mutM}$ and $p_{cspD}$ promoters by PCR, using the $p_{mutM}$- and $p_{cspD}$-specific primers specified in Table 8.

| Table 8 Primers | | | |
|---|---|---|---|
| Promoter of *E. coli* gene | Primer name | Primer sequence (5'-3')* | SEQ ID No.: |
| *mutM* | mutM for | ATTCATATGUGGCGAAAAGTACTAAGTACTTA | 116 |
| | mutM rev | ATCCCCATTACGTGAGGATGGCTAAGGTCCAGTGAA GTACCCTGGGCAAAGAGTTTCATacctt**NCAAGT**AGC ATCTCCAGGAATGAACA | 117 |

(continued)

| Table 8 Primers | | | |
|---|---|---|---|
| Promoter of *E. coli* gene | Primer name | Primer sequence (5'-3')* | SEQ ID No.: |
| *cspD* | cspD fwd | ATTCATATGUCGGTTATCGGCAGAACGCCCTG | 138 |
| | cspD rev | ATCCCCATTACGTGAGGATGGCTAAGGTCCAGTGAA GTACCCTGGGCAAAGAGTTTCATacctt**NCAAGT**GCT TCGACATCCTTCGCAAA | 139 |
| * RBS complementary coding sequences are in bold font; wherein N is either A, G, T or C. The RBS is encoded on the opposite strand of the oligo sequence shown. | | | |

*Chromosomal integration of burden-addiction promoters:* the $p_{mutM}$ and $p_{cspD}$ were individually integrated upstream of the *folP-glmM* gene in the genome of cells of the *E. coli* strain BL21(DE3)pMevT, harboring a lysostaphin producing plasmid pMevT, as follows. Cells of the *E. coli* strain BL21(DE3)pMevT, pre-transformed with the recombineering plasmid pSIM5-tet, were prepared and transformed by electroporation with each of the promoter integration sequences, as described in example 1.2.1. Following the described steps of recombineering and curing of pSIM5-tet, 5 colonies were picked, corresponding to clones with the same promoter integration but having a random variant RBS sequence (see Table 8). Each clone was then transferred to a well of a 96-well plate containing 200 μl 2xYT supplemented with chloramphenicol for maintenance of the plasmid, pMevT, and cultured. 2 μl of each cultured clone was used to validate promotor integration as described in example 1.2.1; prior to freezing the 96-well plate.

*Mevalonic acid production screening:* The *E. coli* strains s19.1.1($p_{cspD}$) and s9.1.4($p_{mutM}$) having the highest mevalonic acid productivity were identified using a screening assay in which strains comprising the four other potential combinations of RBS and promoter were cultured in 96-well plates over six serial passages of 1000x dilution (corresponding to 60 generations) in parallel with the non-burden addicted production *E. coli* strain BL21(DE3)pMevT, as follows. The strains were grown in 200 μL 2xYT media containing 30 mg/L chloramphenicol and 0.5mM IPTG in microtiter plate sealed with breathe-easy seal at 37°C for 21 hours with horizontal shaking. 150 μL of the cultures were mixed with 50 μl 50% glycerol and stored in a 96-well plate at -80°C. Additionally, 2 μl of a 10-fold diluted culture was transferred to 200 μL fresh 2xYT media supplemented with chloramphenicol and 0.5mM IPTG and grown for another 21 hours under identical conditions. In total, 5 transfers identical to the one described above were made and freeze-stocks were made of the overnight cultures with each transfer.

*Mevalonic acid synthesis and detection assay:* The 96-well plate containing freeze-stocks from the second, fifth and sixth transfers were thawed on ice and used to inoculate 10 mL 2xYT with 0.5 mM IPTG and 30 mg/L chloramphenicol and cultivated at 37°C with horizontal shaking (250 rpm) for 54 hours. 300 μL aliquots from each culture were treated with 23μL 20% sulfuric acid; vigorously shaken and then spun down at 13 000 × g for 2 min. Supernatant (medium) samples were injected into an Ultimate 3000 high-performance liquid chromatography running a 5mM sulfuric acid mobile phase (0.6 mL/min) on an Aminex HPX-87H ion exclusion column (300 mm × 7.8 mm, Bio-Rad Laboratories) at 50 °C. A refractive index detector was used for detection. A standard curve for mevalonic acid was generated with mevalonolactone (Sigma-Aldrich) dissolved in 2xYT medium supernatant of a non-producing *E. coli* strain incubated under the same conditions.

4.2 Results

The burden-addicted mevalonic acid-producing strains having the $p_{cspD}$ or $p_{mutM}$ promoters and one of four RBS coding sequence variants controlling expression of the essential genes, *folP-glmM,* were cultivated under simulated large-scale production conditions, achieved by serial passaging. As seen in figure 12, both selected burden-addicted strains s19.1.1($p_{cspD}$) and s9.1.4($p_{mutM}$) retained significantly higher levels of mevalonic acid production as compared to the steep reduction in production seen in the non-burden addicted mevalonic acid-production strain BL21(DE3)pMevT. Since the initial mevalonic acid production rates for each burden-addicted strain was the same and the control BL21(DE3)pMevT strain at seed 0; the smaller decline in production rates for strains s19.1.1($p_{cspD}$) and s9.1.4($p_{mutM}$) is not due to an inherently lower initial burden from mevalonic acid production.

*In summary:* Burden-addiction is demonstrated to enhance long-term stability and production in *E. coli* engineered to synthesize mevalonic acid by coupling essential genes *folP-glmM* transcription to the *E. coli* oxidative stress and glucose starvation sensing promoter $p_{cspD}$, and the heat shock/DNA damage-sensing promoters $p_{mutM}$.

**Example 5: Evaluation of promoters as burden sensors to render yeast cells addicted to the burden of engineered recombinant protein production**

Promoters capable of sensing the burden or load on a cell brought about by recombinant expression of a protein or a biosynthetic pathway, and that then induce the expression of an essential gene, can be used to create a burden addiction genetic circuit tailored for use in yeast. A method for evaluating candidate promoters is illustrated in yeast cells genetically engineered to synthesize recombinant human serum albumin (hSA) or insulin precursor (IP), optionally translationally fused to green fluorescent protein (GFP).

To render growth responsive to the activity of a candidate burden-sensing promoter, the native promoter of an essential gene in the yeast *Saccharomyces cerevisiae* is genetically replaced with a candidate promoter using homologous recombination of linear DNA constructs transformed into the yeast cells using a standard selectable marker. By way of example, candidate promoters may be selected from upregulated promoters of ribosomal RNA genes, such as those transcribed by RNA polymerase I (Laferté et al., 2006), DNA damage sensing promoters (e.g. pOGG1), and unfolded protein response promoters upregulated by the *HAC1* transcription factor (Kimata et al., 2006). In order to ensure that the burden-sensing promoter, once activated, confers the cell with a selective growth advantage compared to a non-sensing promoter, it may be necessary to fine-tune the expression level of essential gene. A range of translational strengths can be engineered by varying the translation initiation region introduced with the burden-sensing promoter.

Clones with potentially different combinations of candidate burden-sensing promoters are selected and evaluated for maintained protein production over 30-100 generations of cell division.

5.1 Materials and methods

*Growth medium:* YPD medium comprises 1% yeast extract, 2% peptone, 2% glucose. SC medium comprises 6.7 g/L yeast nitrogen base without amino acids and with ammonium sulfate, but lacks uracil.

*Chromosomal integration and validation of promoter constructs:* Constructs for chromosomal integration will contain 300-600 bp upstream of the natively regulated gene. Chromosomal integration of the promoter construct is performed by transformation and homologous recombination using standard electroporation procedures for S. *cerevisiae.* Correct chromosomal integration of the promoter was validated using colony PCR.

*Long-term cultivation and production:* A single colony from each strain is transferred to 24-deep well plates and cultivated in 1.8 mL YPD medium under conditions inducing recombinant protein production, at 250 rpm and 30 °C. Following 48 hours of cultivation, cells are passaged to a new deep-well plate under identical conditions by 1000x back-dilution. Samples are analyzed for production using recombinant protein specific assays (e.g. GFP detection) and the cell density is monitored by OD600.

*Growth rate measurements of selected strains:* Growth rates of individual strains are compared to non-burden addicted production strains. The 96-well plate is sealed with breathe-easy film and growth is measured in a Synergy H1 plate reader for 20 hours at 37°C and 754 rpm linear shaking. OD600 is measured every 10 minutes.

5.2 Results

*5.2.1 HAC1-upregulated promoters*

The HAC1-upregulated promoters comprising an unfolded protein response (UPR) element, e.g. *KAR2 (SEQ ID NO.: 91), PDI1 (SEQ ID NO.: 92), SSA1 (SEQ ID NO.: 93)* or *FPR2 (SEQ ID NO.:* 94), are demonstrated to be useful in regulating growth when introduced in front of a native growth regulating gene (e.g. the conditionally essential gene *URA3* encoding orotidine 5'-phosphate decarboxylase essential for pyrimidine biosynthesis) in a recombinant protein production *Saccharomyces* strain producing human insulin precursor or human serum albumin, optionally coupled to GFP. Stability of production is followed under simulated long-term production via serial passages corresponding to 60-80 generations of cell division. In *Saccharomyces* strains comprising the HAC1-upregulated promoter controlling transcription of the growth-regulating gene (URA3), production is expected to be more stable than the corresponding parent recombinant protein production *Saccharomyces* strain.

*5.3.2 RNA polymerase I upregulated promoters*

RNA polymerase I transcribe ribosomal RNA genes in yeast. RNA polymerase I upregulated promoters such as promoters of the genes: *RPL3* (SEQ ID NO.: 95), *RPL6A* (SEQ ID NO.: 96) and *RPL28* (SEQ ID NO.: 97) are useful in regulating growth of yeast essential genes. Such upregulated promoters are introduced in front of native growth regulating gene (e.g. the conditionally essential gene *URA3*) in a recombinant protein overproduction strain producing human insulin precursor or human serum albumin, potentially coupled to GFP. Stability of production is followed by experimentally simulated long-term production via serial passages corresponding to 60-80 generations of cell division. In *Saccharomyces* strains comprising an RNA polymerase I upregulated promoter controlling transcription of the conditionally essential gene, *URA3,* production will be more stable than the corresponding parent recombinant protein production *Saccharomyces* strain.

5.3.3 *DNA damage responsive promoters*

DNA damage response in yeast, resulting from heterologous expression broadly induces transcription of the DNA repair system, including *OGG1 (SEQ ID NO.: 98), RAD51 (SEQ ID NO.:* 99) and *RAD54 (SEQ ID NO.: 100).* Promoters of the genes encoding *OGG1, RAD51* or *RAD54,* when operatively linked to an essential gene, are useful for regulating growth of a yeast production cell of the invention. Such promoters are introduced in front of native essential gene (e.g. the growth regulating gene encoding URA3) in cells of a yeast protein production strain producing human insulin precursor or human serum albumin, optionally fused to GFP.

Stability of production is followed by under simulated long-term production via serial passages corresponding to 60-80 generations of cell division. In strains with a $p_{RAD51}$ and/or $p_{RAD54}$-upregulated essential gene, production will be more stable.

*In summary:* Burden addiction enhances long-term stability and production in cells of budding yeast engineered to synthesize human serum albumin production or insulin precursor by coupling essential gene transcription to the burden-sensing promoters selected from promoters activated during burden of recombinant protein production or associated with ribosomal RNA promoters (Table 2).

**Example 6 Examples of burden-addicted yeast strains producing human serum albumin showing enhanced long-term production stability**

Burden-addiction systems for use in yeast strains include promoters derived from genes encoding: 1) Protein isomerase PDI1 encoding a chaperone belonging to the unfolded protein response of yeasts such as S. *cerevisiae* and *P. pastoris,* and whose abundance is frequently upregulated in response to overexpression of a recombinant protein; 2) Ribosomal subunits encoded by the *RPL6A* and *RPL3* genes and 3) *FPR2* encoding a peptidyl-prolyl cis-trans isomerase which is known to be activated upon DNA replication stress. Burden-addiction systems based on these promoters were introduced into a strain of *Pichia pastoris* engineered to express and secrete human serum albumin in order to determine their effect on long term human serum albumin (hSA) production stability.

6.1 Materials and methods:

The *Pichia pastoris* (*Komagataella phaffii*) strain EGS31 is a derivative of the CBS7435 strain (NRRL-Y11430 or ATCC 76273), engineered to secrete hSA by a genomically integrated cDNA version of the hSA coding *ALB1* gene under control of the *AOX1* promoter. *Strain construction:* Burden-addicted versions of the EGS31 strain were generated by genetically integrating a construct comprising a kanMX conditionally selectable G418 resistance gene operably linked to one of the burden-responsive promoters: *pPDI1, pFPR2, pRPL3* and *pRPL6A.* These burden-addiction constructs were integrated into the KU70 genomic locus of the *P. pastoris* strain by transforming EGS31 cells with linear integration DNA (sequences N1-N3 respectively) flanked by >750 bp homology arms. Transformation was performed using a standard electroporation procedure on exponentially grown cells pretreated with lithium acetate and dithiothreitol (Wu & Letchworth, 2004).

**Table: Recombinant genes and burden-addiction constructs introduced into *P. pastoris***

| Gene/construct name | Construct nucleotide SEQ ID NO: | Encoded protein SEQ ID NO.: |
|---|---|---|
| pAOX1-ALB construct | SEQ ID NO: 155 | hSA protein SEQ ID NO: 156 |
| KU70(up)-pPDI1-kanMX-terminator-KU70(dw) | SEQ ID NO: 157 | kanMX protein SEQ ID NO: 158 |
| KU70(up)-pFPR2-kanMX-terminator-KU70(dw) | SEQ ID NO: 159 | kanMX protein SEQ ID NO: 160 |
| KU70(up)-pRPL3-kanMX-terminator-KU70(dw) | SEQ ID NO: 161 | kanMX protein SEQ ID NO: 162 |

(continued)

| Gene/construct name | Construct nucleotide SEQ ID NO: | Encoded protein SEQ ID NO.: |
| --- | --- | --- |
| KU70(up)-pRPL6A-kanMX-terminator-KU70(dw) | SEQ ID NO: 163 | kanMX protein SEQ ID NO: 164 |

*Growth Media:* BMGY and BMMY liquid media (1L) was prepared as follows: 10 g yeast extract and 20 g peptone was added to 700mL $H_2O$; mixed with a magnetic stirrer and then autoclaved. After cooling to room temperature; the following was added to the solution: 100ml 1M potassium phosphate buffer (pH 6.0); 100 ml 13.4% (w/v) Yeast Nitrogen Base with Ammonium Sulfate without amino acids; 2 ml 0.02% (w/v) biotin, and in the case of BMGY, 100 ml 10% (v/v) glycerol was added; and in the case of BMMY, 100mL 5% (v/v) methanol was added.

*Cultivation: EGS31* and burden-addicted EGS31 strains were streaked on YPD (1 % yeast extract, 2 % peptone, 2 % D-glucose) agar plates and incubated overnight at 30 deg. C. Single colonies were picked and pre-cultured in 2 mL BMGY where cultures of burden-addicted strains were supplemented 50 ug/mL G418 and cultured at 30 deg. C with 300 rpm horizontal shaking overnight. Expression cultures were seeded using 1 uL preculture into 500 $\mu$L BMMY medium containing different concentrations (0 $\mu$g/mL, 750 $\mu$g/mL) of G418 in a 96-well deep well plate with aerating lid to generate "seed 1".

The cultures were incubated at 30 deg. C for 72 hours (300 rpm horizontal shaking). To generate the next seed, four additional times, grown cultures were serially passaged (500x dilution) to new 500 $\mu$L BMMY medium containing different concentrations (0 $\mu$g/mL, 750 $\mu$g/mL) of G418 in a 96-well deep well plate with aerating lids. At each serial passage, glycerol stocks (20 % glycerol) were stored at -80 deg. C from the grown cultures.

To quantify production of secreted hSA, quantification cultures were re-grown from glycerol stocks in 500 $\mu$L BMMY medium containing different concentrations (0 $\mu$g/mL and 750 $\mu$g/mL) of G418 in a 96-well deep well plate with aerating lid for 72 hours.

Cultures were centrifuged at 3000 g for 15 minutes and the concentration in 50 $\mu$L supernatants was quantified using a hSA-specific ELISA kit (Abcam catalog no: ab179887: Human Albumin SimpleStep ELISA® Kit) following manufacturer's instructions.

6.2 Results

Strains with a genomically integrated burden addiction promoter, *PDI1,* operably linked to the selectable *kanMX* gene displayed higher production of secreted hSA when strain's burden-addiction system was activated by addition of G418 (figure 13). Further, improved hSA production was seen with higher selection (750 $\mu$g/mL G418), for each of the other tested burden-addiction promoters after approximately 30 cell divisions (Figure 14A - Seed 1). When further cultivated for about an additional 10 cell divisions (Figure 14B - Seed 2) the strains show enhanced levels of hAS production when burden addiction is activated (150-750 $\mu$g/mL G418) compared to the non burden-addicted control and strains without burden addiction activated (0 $\mu$g/mL G418).

*In conclusion,* the cultivation of yeast cells comprising the exemplified burden-addiction systems of the invention, under conditions that activate their respective burden-addiction system, is believed to enrich for high-producing yeast variants within the cultured population. While an increase in hSA production may be detectable after relatively short cultivation (30 cell divisions), enrichment for high-producing yeast variants is both maintained and further enhanced over longer cultivation periods when traditional cultures commonly exhibit a significant decline in productivity.

**Example 7 Identification of suitable burden sensing promoter candidates**

Different engineered production genes and pathways elicit different transcriptional responses indicative of the production process. In order to identify suitable promoter candidates for use as burden sensors, the following experiment was conducted.

*Methods:* Typical genetic escaper cells were isolated from long-term cultivation with the genetically engineered microbial production cells of interest in the intended fermentation medium. Suitable genetic escaper cells are characterized by having at least 5 % higher exponential-phase growth rate and at least 30 % lower production rate or product yield than the original genetically engineered production cell.

Production cells and corresponding escaper cells were cultured under intended fermentation conditions, scaled down conditions or shake flask conditions mimicking intended fermentation conditions. At time points corresponding to the highest rate of production in the production cells, samples were taken for RNA sequencing. Total RNA was purified using Purelink RNA Mini kit (Thermo Fischer) and prepared using TruSeq Stranded mRNA kit (Illumina) following the kit manufacturer's instructions. Reads were mapped and analyzed to the reference genome of the strain and next analyzed for differential expression between the production cells and corresponding escaper cells.

*Results:* Candidate suitable promoters were identified as those driving expression of genes that showed a differential expression of >3 fold higher expression in the production organism relative to at least one isolated genetic escape strain.

**References**

Bonde, M. T., Pedersen, M., Klausen, M. S., Jensen, S. I., Wulff, T., Harrison, S., et al. (2016). Predictable tuning of protein expression in bacteria. Nat. Methods 13. doi: 10.1038/nmeth.3727.

Koskiniemi, S., Pränting, M., Gullberg, E., Näsvall, J., & Andersson, D. I. (2011). Activation of cryptic aminoglycoside resistance in Salmonella enterica. Molecular Microbiology, Vol. 80, pp. 1464-1478. https://doi.org/10.1111/j.1365-2958.2011.07657.x

Giaever G et al., Nature. 2002 Jul 25;418(6896):387-91. DOI: 10.1038/nature00935 Jain, R., Kumar, P. & Varshney, U. A distinct role of formamidopyrimidine DNA glycosylase (MutM) in down-regulation of accumulation of G, C mutations and protection against oxidative stress in mycobacteria. DNA Repair (Amst). 6, 1774-1785 (2007).

Kimata, Y., Ishiwata-Kimata, Y., Yamada, S., and Kohno, K. (2006). Yeast unfolded protein response pathway regulates expression of genes for anti-oxidative stress and for cell surface proteins. Genes to Cells 11, 59-69. doi:10.1111/j.1365-2443.2005.00921.x.

Laferté, A., Favry, E., Sentenac, A., Riva, M., Carles, C., and Chédin, S. (2006). The transcriptional activity of RNA polymerase I is a key determinant for the level of all ribosome components. Genes Dev. 20, 2030-2040. doi:10.1101/gad.386106.

Maeda, M., Shimada, T., and Ishihama, A. (2015). Strength and Regulation of Seven rRNA Promoters in Escherichia coli. PLoS One 10, 1-19. doi:10.1371/journal.pone.0144697.

Nonaka, G., Blankschien, M., Herman, C., Gross, C. a, and Rhodius, V. a (2006). Regulon and promoter analysis of the E. coli heat-shock factor, sigma32, reveals a multifaceted cellular response to heat stress. Genes Dev. 20, 1776-89. doi:10.1101/gad.1428206.

Pitera, D. J., Paddon, C. J., Newman, J. D., and Keasling, J. D. (2007). Balancing a heterologous mevalonate pathway for improved isoprenoid production in Escherichia coli. Metab. Eng. 9, 193-207. doi:10.1016/j.ymben.2006.11.002.

Rugbjerg, P., Sarup-Lytzen, K., Nagy, M., and Sommer, M. O. A. (2018). Synthetic addiction extends the productive life time of engineered Escherichia coli populations. Proc. Natl. Acad. Sci. 115, 2347-2352. doi:10.1073/pnas.1718622115.

Wu, S. and Letchworth, G.J. (2004) High efficiency transformation by electroporation of Pichia pastoris pretreated with lithium acetate and dithiothreitol. Biotechniques 36, 152-154

Yoon, S. H., Han, M. J., Lee, S. Y., Jeong, K. J., and Yoo, J. S. (2003). Combined transcriptome and proteome analysis of Escherichia coli during high cell density culture. Biotechnol. Bioeng. 81, 753-767. doi:10.1002/bit.10626.

Uppal, S., Shetty, D. M., & Jawali, N. (2014). Cyclic AMP receptor protein regulates cspd, a bacterial toxin gene, in Escherichia coli. Journal of Bacteriology, 196(8), 1569-1577. https://doi.org/10.1128/JB.01476-13

Yamanaka, K., & Inouye, M. (1997). Growth-phase-dependent expression of cspD, encoding a member of the CspA family in Escherichia coli. Journal of Bacteriology, 179(16), 5126-5130. https://doi.org/10.1128/jb.179.16.5126-5130.1997

**Claims**

1. A microbial production cell genetically engineered to synthesize a product, said microbial cell further comprising:

   a) an essential gene operably linked to a burden-sensing promoter and a synthetic Ribosomal Binding Site (RBS), wherein the sequence of said synthetic RBS is selected to modify the translational strength of the essential gene; and enable the essential gene to regulate the growth rate of a cell;
   wherein said essential gene is either a non-conditional essential gene wherein expression of the gene is essential for cell growth and/or survival irrespective of the composition of the growth medium, or conditionally essential wherein the composition of the production cell's growth medium must be adjusted to lack specific nutrients or supplemented with growth inhibitors, wherein knock-out of said non-conditional essential gene will result in loss of cell viability or failure to grow, irrespective of growth conditions; while knockout of the conditional essential gene will result in a failure to grow or loss of cell viability depending on the composition of the medium;

wherein said burden-sensing promoter is heterologous with respect to said essential gene;

wherein synthesis of the product confers a burden on said cell;

wherein said burden, conferred by synthesis of said product, confers a fitness cost measured as a percent reduction in the exponential-phase growth rate of the microbial production cell relative to a corresponding non-producing microbial cell selected from among $\geq$ 5 %, $\geq$10%, $\geq$15%, $\geq$20%, $\geq$25%, $\geq$35% and $\geq$ 45 % when said essential gene in said production cell and in said non-producing cell is operably linked to its native promoter; wherein expression of said essential gene is up-regulated when said burden-sensing promoter is induced by said burden relative to a basal level expression of said essential gene when said burden-sensing promoter is not induced; wherein said burden-sensing promoter is induced by said burden and up-regulates said essential gene in said production microbial cell when cultured under production conditions as compared to a mutant derivative of said microbial cell producing at least 50% less of the product.

2.  The microbial production cell according to claim 1, wherein said cell comprises

    b) one or more genes encoding the product or encoding a metabolic pathway for synthesis of the product, wherein said one or more genes are operably linked to a promoter.

3.  The microbial production cell according to claim 1 or 2,
    wherein the burden-sensing promoter is induced by the burden in the production cell resulting from production of the product and not by the product itself.

4.  The microbial production cell according to any one of claims 1-3, wherein the cell is:

    i) a bacterium belonging to a genus selected from among *Escherichia, Lactobacillus, Lactococcus, Corynebacterium, Bacillus, Acetobacter, Acinetobacter, Pseudomonas; Proprionibacterium, Bacteroides,* and *Bifidobacterium;* or
    ii) a yeast belonging to a genus selected from among *Saccharomyces, Kluyveromyces, Candida, Pichia, Komagataella, Cryptococcus, Debaromyces, Hansenula, Yarrowia, Zygosaccharomyces* and *Schizosaccharomyces;* or
    iii) a filamentous fungus selected from among *Penicillium, Rhizopus, Fusarium, Fusidium, Gibberella, Mucor, Mortierella, Trichoderma Thermomyces, Streptomyces* and *Aspergillus.*

5.  The microbial production cell according to any one of claims 1-4, wherein the essential gene is a non-conditional essential gene.

6.  The microbial production cell according to any one of claims 1-5, wherein the cell is a bacterium and the essential gene is an *E. coli* gene or operon selected from among *folP-glmM, glmM, murI, asd, thyA, usA, rpoD, nusG, rpsU, accD, degS, fldA, ftsN, hflB, lolA, mraY, mreD, murA, murB, murF, nadD, rplV* and *rpsG,* or a homologue thereof.

7.  The microbial production cell according to any one of claims 1-6, wherein the essential gene is operably linked to a synthetic RBS whose sequence is selected to modify the translational strength of the essential gene independent of induction of said burden-sensing promoter.

8.  The microbial production cell according to any one of claims 1-7, wherein the burden-sensing promoter is selected from among:

    i) a $\sigma$ factor regulated promoter, such as a $\sigma^{32}$, $\sigma^{B}$ or $\sigma^{S}$ factor regulated promoter,
    ii) a ribosomal RNA promoter,
    iii) an HAC1-upregulated promoter comprising a UPR element, and
    iv) a DNA damage-sensing promoter.

9.  The microbial production cell according to any one of claims 1-8, wherein the cell is **characterized by** an increased product yield after at least 25, 30, 35, 40, 45, 50, 60, 70, 80, 90 or 100 generations of cell division from a single cell, as compared to a parent microbial production cell lacking said essential gene operably linked to a burden-sensing promoter.

10. The microbial production cell according to any one of claims 1-9, wherein the cell is **characterized by** an increased product yield of at least 10, 25, 50, or 80% following at least 50 generations of cell division from a single cell as compared to a parent microbial production cell lacking said essential gene operably linked to a burden-sensing promoter.

11. A method of product biosynthesis comprising the steps of:

i) providing a microbial production cell according to any one of claims 1-10,
ii) introducing the cell into a cultivation medium comprising substrate for production of said product, and
iii) recovering said product.

12. A method of product biosynthesis according to claim 11, wherein the product is selected from among an: amino acid, organic acid, terpenoid, isoprenoid, polyketide, alcohol, sugar, vitamin, aldehyde, carboxylic acid, fatty acid, peptide, enzyme, therapeutic protein and precursor thereof, human growth hormone, insulin, glucagon-like peptide-1, monoclonal and polyclonal antibody, and single-fragment antibody.

13. Use of an essential gene operably linked to a burden-sensing promoter and a synthetic RBS to enhance product yield of a cultured population of microbial production cells arising from a single cell,

wherein said essential gene is either a non-conditional essential gene wherein expression of the gene is essential for cell growth and/or survival irrespective of the composition of the growth medium, or conditionally essential wherein the composition of the production cell's growth medium must be adjusted to lack specific nutrients or supplemented with growth inhibitors, wherein knock-out of said non-conditional essential gene will result in loss of cell viability or failure to grow, irrespective of growth conditions; while knockout of the conditional essential gene will result in a failure to grow or loss of cell viability depending on the composition of the medium, wherein said burden-sensing promoter is heterologous with respect to said essential gene,
wherein the sequence of said synthetic RBS is selected to modify the translational strength of the essential gene; and enable the essential gene to regulate the growth rate of a cell,
wherein production of the product confers a burden on said microbial production cell,
wherein said burden, conferred by synthesis of said product, has a fitness cost measured as a percent reduction in the exponential phase growth rate of the microbial production cell relative to a corresponding non-producing microbial cell selected from among $\geq 5\%$, $\geq 10\%$, $\geq 15\%$, $\geq 20\%$, $\geq 25\%$, $\geq 35\%$ and $\geq 45\%$ when said essential gene in said production cell and in said non-producing cell is operably linked to its native promoter,
wherein expression of said essential gene is up-regulated when said burden-sensing promoter is induced by said burden relative to a basal level expression of said essential gene when said burden-sensing promoter is not induced, and
wherein said burden-sensing promoter is induced by said burden and up-regulates said essential gene in said production microbial cell when cultured under production conditions as compared to a mutant derivative of said microbial cell producing at least 50% less of the product.

14. The use of an essential gene operably linked to a burden-sensing promoter according to claim 13, wherein the burden-sensing promoter is induced by the burden in the production cell resulting from production of the product and not by the product itself.

15. Use of a microbial production cell according to any one of claims 1-10 for producing a biosynthetic product.

16. The use of a microbial production cell according to claim 15, wherein the product is selected from among an: amino acid, organic acid, terpenoid, isoprenoid, polyketide, alcohol, sugar, vitamin, aldehyde, carboxylic acid, fatty acid, peptide, enzyme, therapeutic protein and precursor thereof, human growth hormone, insulin, glucagon-like peptide-1, monoclonal and polyclonal antibody, and single-fragment antibody.

**Figure 1**

**Figure 2**

**Figure 3**

**Figure 4**

**Figure 5**

**Figure 6**

**Figure 7**

**Figure 8**

**Figure 9**

**Figure 10**

**Figure 11**

**Figure 12**

**Figure 13**

**Figure 14 A**

Seed 1

**Figure 14 B**

Seed 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Gene Essentiality - Method and Protocols*, January 2015
- **BONDE, M. T** ; **PEDERSEN, M.** ; **KLAUSEN, M. S** ; **JENSEN, S. I** ; **WULFF, T** ; **HARRISON, S et al.** Predictable tuning of protein expression in bacteria.. *Nat. Methods*, 2016, vol. 13
- **KOSKINIEMI, S** ; **PRÄNTING, M.** ; **GULLBERG, E.** ; **NÄSVALL, J.** ; **ANDERSSON, D. I.** Activation of cryptic aminoglycoside resistance in Salmonella enterica.. *Molecular Microbiology*, 2011, vol. 80, 1464-1478, https://doi.org/10.1111/j.1365-2958.2011.07657.x
- **GIAEVER G et al.** *Nature*, 25 July 2002, vol. 418 (6896), 387-91
- **JAIN, R.** ; **KUMAR, P** ; **VARSHNEY, U.** A distinct role of formamidopyrimidine DNA glycosylase (MutM) in down-regulation of accumulation of G, C mutations and protection against oxidative stress in mycobacteria.. *DNA Repair (Amst).*, 2007, vol. 6, 1774-1785
- **KIMATA, Y** ; **ISHIWATA-KIMATA, Y** ; **YAMADA, S** ; **KOHNO, K.** Yeast unfolded protein response pathway regulates expression of genes for anti-oxidative stress and for cell surface proteins.. *Genes to Cells*, 2006, vol. 11, 59-69
- **LAFERTÉ, A.** ; **FAVRY, E** ; **SENTENAC, A.** ; **RIVA, M.** ; **CARLES, C** ; **CHÉDIN, S**. The transcriptional activity of RNA polymerase I is a key determinant for the level of all ribosome components.. *Genes Dev.*, 2006, vol. 20, 2030-2040
- **MAEDA, M** ; **SHIMADA, T.** ; **ISHIHAMA, A**. Strength and Regulation of Seven rRNA Promoters in Escherichia coli.. *PLoS One*, 2015, vol. 10, 1-19

- **NONAKA, G** ; **BLANKSCHIEN, M.** ; **HERMAN, C** ; **GROSS, C. A** ; **RHODIUS, V. A**. Regulon and promoter analysis of the E. coli heat-shock factor, sigma32, reveals a multifaceted cellular response to heat stress.. *Genes Dev.*, 2006, vol. 20, 1776-89
- **PITERA, D. J** ; **PADDON, C. J.** ; **NEWMAN, J. D** ; **KEASLING, J. D.** Balancing a heterologous mevalonate pathway for improved isoprenoid production. *Escherichia coli. Metab. Eng.*, 2007, vol. 9, 193-207
- **RUGBJERG, P** ; **SARUP-LYTZEN, K.** ; **NAGY, M.** ; **SOMMER, M. O. A**. Synthetic addiction extends the productive life time of engineered Escherichia coli populations.. *Proc. Natl. Acad. Sci.*, 2018, vol. 115, 2347-2352
- **WU, S.** ; **LETCHWORTH, G.J.** High efficiency transformation by electroporation of Pichia pastoris pretreated with lithium acetate and dithiothreitol.. *Biotechniques*, 2004, vol. 36, 152-154
- **YOON, S. H.** ; **HAN, M. J.** ; **LEE, S. Y** ; **JEONG, K. J.** ; **YOO, J. S**. Combined transcriptome and proteome analysis of Escherichia coli during high cell density culture.. *Biotechnol. Bioeng.*, 2003, vol. 81, 753-767
- **UPPAL, S** ; **SHETTY, D. M** ; **JAWALI, N.** Cyclic AMP receptor protein regulates cspd, a bacterial toxin gene. *Escherichia coli. Journal of Bacteriology*, 2014, vol. 196 (8), 1569-1577, https://doi.org/10.1128/JB.01476-13
- **YAMANAKA, K** ; **INOUYE, M.** Growth-phase-dependent expression of cspD, encoding a member of the CspA family in Escherichia coli.. *Journal of Bacteriology*, 1997, vol. 179 (16), 5126-5130, https://doi.org/10.1128/jb.179.16.5126-5130.1997